(19) Europäisches Patentamt
European Patent Office
Office européen des brevets

(11) Numéro de publication: **0 069 018**
**B1**

(12) # FASCICULE DE BREVET EUROPEEN

(45) Date de publication du fascicule du brevet: **26.02.86**

(51) Int. Cl.[4]: **C 07 D 233/64**, C 07 B 59/00, G 01 N 33/534, C 07 C 69/712, C 07 C 59/68, A 61 K 39/385

(21) Numéro de dépôt: **82401188.6**

(22) Date de dépôt: **28.06.82**

(54) **Dérivés de stilbène radioactifs marqués à l'iode, procédé et intermédiaires de préparation, et leur application aux dosages radioimmunologiques.**

(30) Priorité: **01.07.81 FR 8112934**

(43) Date de publication de la demande: **05.01.83 Bulletin 83/1**

(45) Mention de la délivrance du brevet: *26.02.86 Bulletin 86/9*

(84) Etats contractants désignés: **AT BE CH DE GB IT LI NL SE**

(56) Documents cités:
**JOURNAL OF LABELLED COMPOUNDS AND RADIOPHARMACEUTICALS, vol. 16, no. 3, 1979, Chichester, GB, H.J. JOHNSON et al.: "Preparation of the radioiodinated histamine amide of 4-0-(carboxypropyl)-diethylstilbestrol, pages 501-506**
**ANNALS OF CLINICAL AND LABORATORY SCIENCE, vol. 7, no. 1, 1977, Philadelphia, US, R.M. GUTIERREZ-CERNOSEK et al.: "Radioimmunoassays for monitoring exposure to potential carcinogens" pages 35-41**
**CHEMICAL ABSTRACTS, vol. 95, no. 13, 28 septembre 1981, réf.: 113533f, page, 529, Columbus, Ohio, US, D.J. HARWOOD et al.: "A radioimmunoassay method for the measurement of residues of the anabolic agent, hexestrol, in tissues of cattle and sheep"**

(73) Titulaire: **ROUSSEL-UCLAF, 35, boulevard des Invalides, F-75007 Paris (FR)**

(72) Inventeur: **Jouquey, Alain, 137, rue des Pyrénées, F-75020-Paris (FR)**
Inventeur: **Touyer, Gaetan, Tour Giralda 2, square Vitrune, F-75020-Paris (FR)**

(74) Mandataire: **Bourgouin, André et al, ROUSSEL-UCLAF 111, route de Noisy Boîte postale no 9, F-93230 Romainville (FR)**

(56) Documents cités: (suite)
**Journal Vet. Pharmacol. Therap. (1980), 3(4), pp. 245-254 (Eng.)**

**Le dossier contient des informations techniques présentées postérieurement au dépôt de la demande et ne figurant pas dans le présent fascicule.**

**EP 0 069 018 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention a pour objet de nouveaux dérives de stilbène radioactifs marqués à l'iode, leur procédé et les intermédiaires de préparation et leur application aux dosages radioimmunologiques.

Elle a pour objet les dérivés de stilbene radioactifs marquée à l'iode 125 ou 131, de formule générale I:

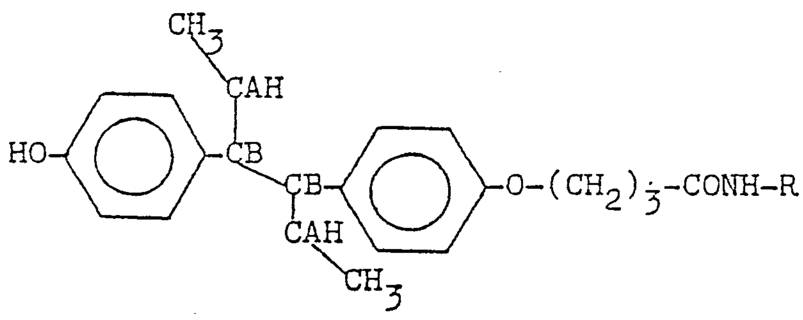

dans laquelle A et B représentent chacun un atome d'hydrogène ou représentent ensemble une double liaison et R représente le reste d'un acide aminé $RNH_2$ ou le reste d'un dérivé de ce dernier, ce reste possédant un groupe accepteur d'iode et étant marqué à l'iode 125 ou 131.

Par dérivé d'un acide aminé, on entend de préférence le dérivé décarboxylé d'un acide aminé ou un ester d'alcoyle inférieur dudit acide aminé.

On connaissait déjà des dérivés de stilbènes radioactifs et leur utilisation dans le dosage radioimmunologique.

Ainsi un amide formé avec l'histamine et le 4-0-(carboxy-propyl) diéthylstilbestrol, substitué sur le reste histamine par l'iode 125 est décrit par H.J. Johnson, S.F. Cernosek et R.M. Gutierrez-Cernosek dans Journal of Labelled Compounds and Radiopharmaceuticals. Vol. XVI, No. 3 p. 501-506.

Dans ce document, le composé est mal défini et, de plus, il permet d'envisager uniquement le dosage radioimmunologique du diéthylstilbestrol. Enfin, il ne prévoit pas le dosage d'autres stilbènes tels que le diénestrol ou l'hexestrol.

La publication de J. Vet. Pharmacol. Ther. 1980, 3, p. 245-54, décrit une méthode de dosage de résidus d'hexestrol dans les tissus et les liquides biologiques des animaux d'élevage. Ce dosage est effectué au moyen d'hexestrol radioactif marqué au tritium.

La méthode de cette publication permet donc seulement le dosage de l'hexestrol.

Par contre, les composés de formule I de la présente invention permettent, avec une grande précision, non seulement le dosage du diénestrol et de l'hexestrol, mais également celui du diéthylstilbestrol.

L'invention a plus particulièrement pour objet

les derives de stilbène radioactifs marques à l'iode de formule générale I, dans laquelle R represente le reste d'un acide amine $RNH_2$ ou le reste d'un dérive de ce dernier, choisi dans le groupe constitué par l'histidine, la tyrosine, l'histamine, la tyramine et le tyrosinate de methyle, marqués à l'iode 125 ou 131.

Parmi les produits de formule generale I, on citera notamment:

- le dienestrol 4-0-monocarboxypropyl, couplé à 1' ($^{125}I$) histamine de formule:

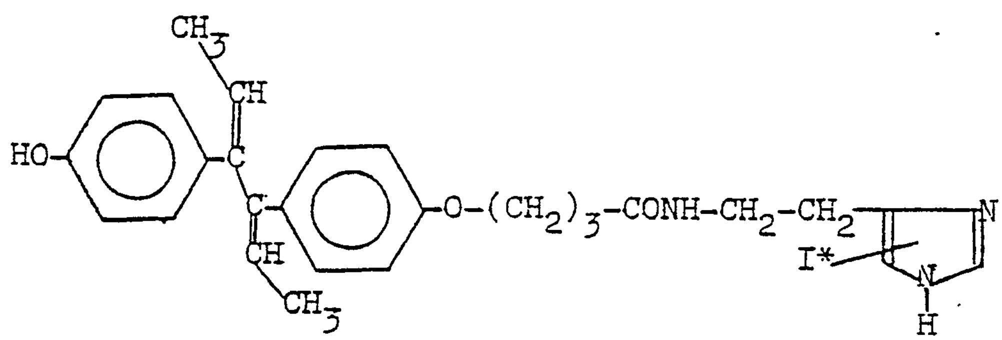

dans laquelle le signe * indique qu'il s'agit d'iode $^{125}$ radicactif, lequel iode $^{125}$ peut être en position 2 ou 5;

- l'hexestrol 4-0-monocarboxypropyle, couplé a l' ($^{125}$ I) histamine de formule

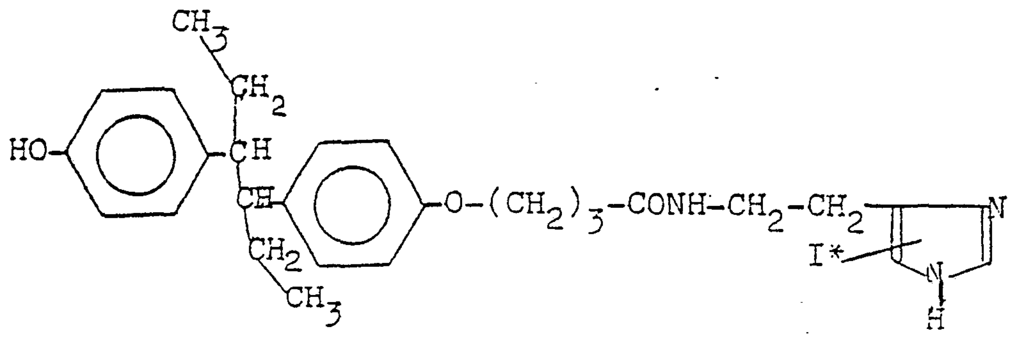

dans laquelle le signe * indique qu'il s'agit d'iode $^{125}$ radioactif, lequel iode $^{125}$ peut être en position 2 ou 5.

L'invention a également pour objet, un procédé de préparation des produits répondant à la formule générale I ci-dessus; ce procédé est caractérisé en ce que:

- on fait réagir un produit de formule générale II:

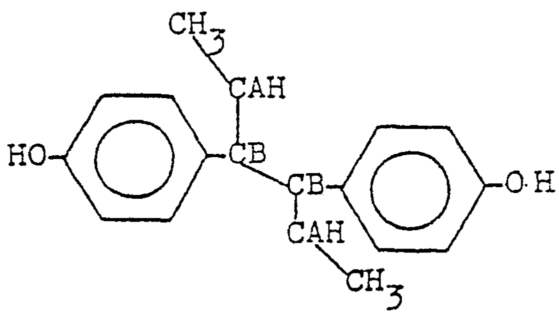

dans laquelle et dans ce qui suit, A et B ont les significations précitées, avec un halogéno butyrate d'alcoyle, en présence d'une base, pour obtenir l'ether de formule générale III:

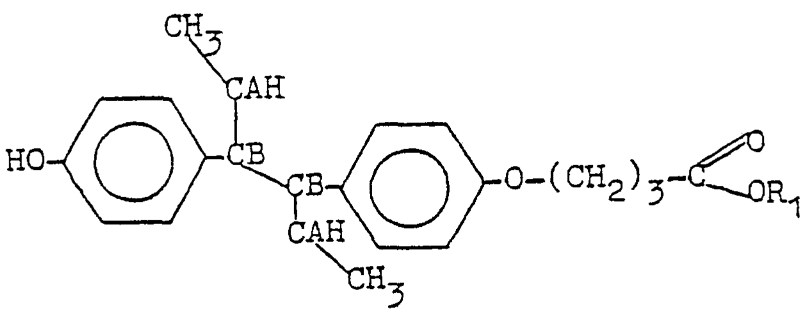

dans laquelle $R_1$ représente un radical alcoyle ayant au plus 6 atomes de carbone,
- dont on saponifie la fonction ester, pour obtenir un produit de produit de formule générale IV:

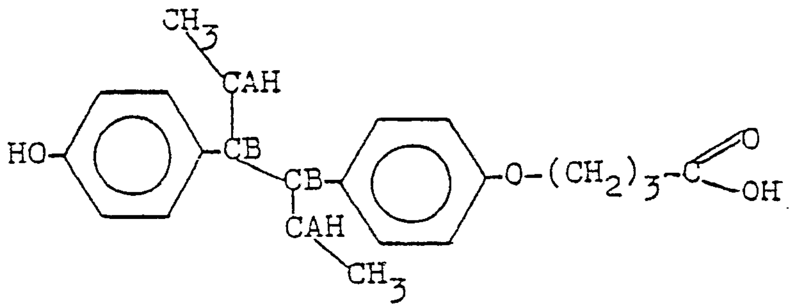

- puis fixé sur la fonction acide de ce produit de formule IV un groupement activateur de la fonction carbonyle et obtient le produit de formule générale V:

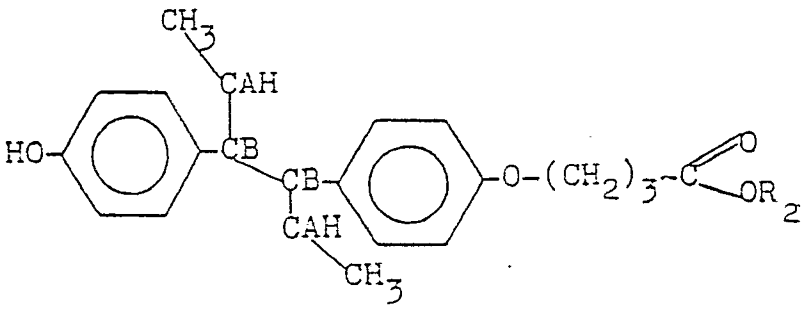

dans laquelle $R_2$ représente un groupement activateur de la fonction carbonyle,
- que l'on fait réagir avec un acide amine accepteur d'iode ou avec un dérivé d'un tel acide et obtient le produit cherché de formule générale I.

Ce procédé est encore caractérisé en ce que:
- l'acide aminé ou le dérivé d'acide aminé accepteur d'iode est choisi dans le groupe constitué par l'histidine, la tyrosine, l'histamine, la tyramine et le tyrosinate de méthyle,
- on fixé sur la fonction acide du produit de formule générale IV un groupement activateur de la fonction

carbonyle, en faisant réagir le produit de formule IV avec un halogénoformiate d'alcoyle, en présence d'une base tertiaire, en milieu anhydre et sous atmosphère inerte et obtient le produit de formule générale V:

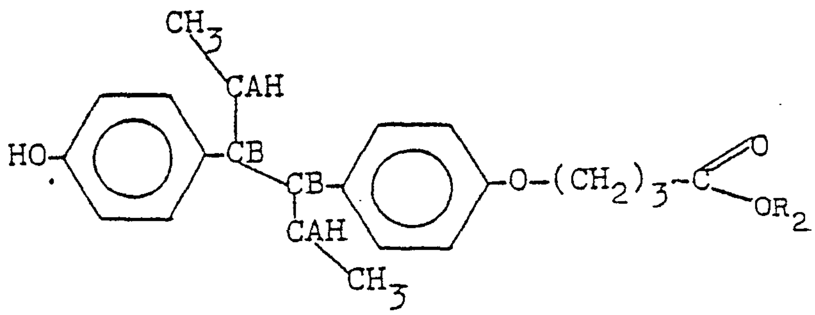

dans laquelle $R_2$ est un groupement activateur de la fonction carbonyle, de formule -CO-O-Alk, Alk étant un radical alcoyle ayant au plus 6 atomes de carbone.

Dans les conditions préférentielles de mise en oeuvre, le procédé de l'invention est caractérisé par les points suivants:

- l'halogénobutyrate d'alcoyle que l'on fait réagir sur le produit de formule générale II est le bromobutyrate d'éthyle et on opère en présence de carbonate de potassium;
- on saponifie la fonction ester du composé de formule générale III, au moyen de la soude méthanolique;
- le produit permettant de fixer un groupement activateur de la fonction carbonyle que l'on fait réagir avec le produit de formule générale IV est le chloroformiate d'isobutyle et l'on opère en présence de tri-n-butylamine, dans le dioxane anhydre et sous atmosphère inerte;
- l'acide aminé ou son dérivé que l'on fait réagir avec le produit de formule générale V est l'histamine marquée à l'iode 125 ou 131 et l'on opère sous atmosphère inerte.

L'activation de la fonction carbonyle du produit de formule générale IV peut aussi être réalisée en faisant agir sur celui-ci le N-hydroxy succinimide ou le dicyclohexylcarbodiimide, générateur des groupements activateurs de ladite fonction carbonyle.

Le procédé décrit permet d'obtenir au départ du diénestrol:

- 1e dienestrol 4-0-monocarboxypropyl couplé à 1'($^{125}$I)-histamine de formule

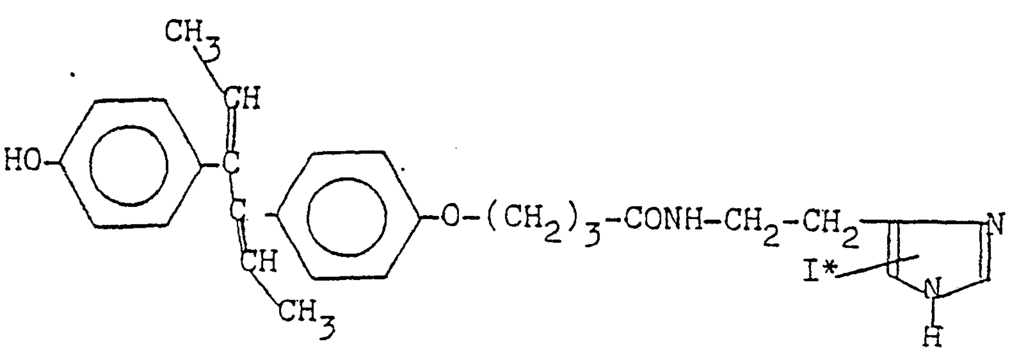

dans laquelle le signe * indique qu'il s'agit d'iode[125] radioactif, lequel iode [125] peut être en position 2 ou 5.

- au départ de l'hexestrol:
- l'hexestrol 4-0-monocarboxypropyl couplé à l'($^{125}$I)-histamine de formule

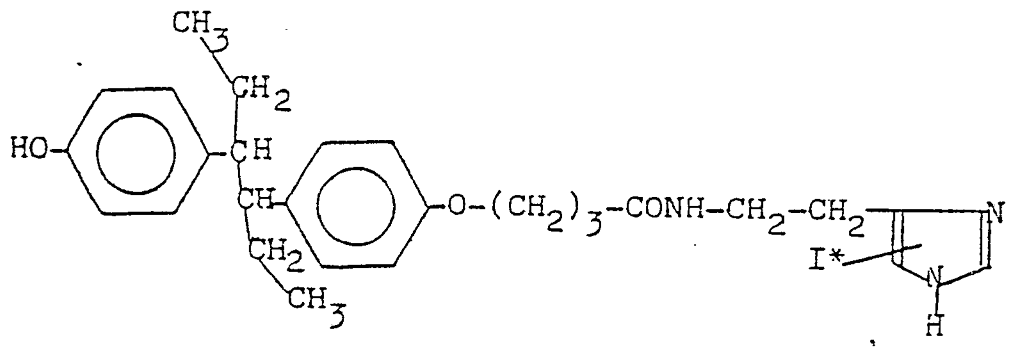

dans laquelle le signe * indique qu'il s'agit d'iode [125] radioactif, lequel iode [125] peut être en position 2 ou 5.

L'invention a également pour objet l'application des produits de formule générale I, aux dosages radioimmunologiques du diénestrol, de l'hexestrol ainsi que du diéthylstilbestrol, dans la bile, l'urine les feces, le plasma et les tissus des animaux ou de l'homme, ainsi que dans les aliments des animaux ou de l'homme.

L'invention a également pour objet à titre de produits industriels nouveaux et notamment à titre de produits intermédiaires nécessaires à l'exécution du procédé de l'invention, les produits de formule générale VI:

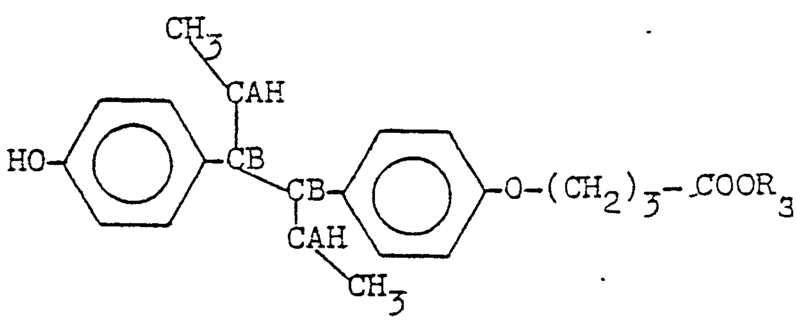

dans laquelle A et B représentent chacun un atome d'hydrogène ou représentent ensemble une double liaison et $R_3$ représente un atome d'hydrogène, un radical alcoyle ayant au plus 6 atomes de carbone ou un groupement activateur de la fonction carbonyle.

Comme indique precédemment, ce groupement activateur de la fonction carbonyle peut être choisi dans le groupe constitué par le groupement -CO-O-Alk, le radical Alk étant un radical alcoyle ayant au plus 6 atomes de carbone, ou les groupements résultant de l'action du N-hydroxy succinimide ou du dicyclohexylcarbodiimide sur la fonction carbonyle.

Parmi ces produits, on citera les produits suivants:

- le dienestrol 4-0-monocarbéthoxypropyl de formule

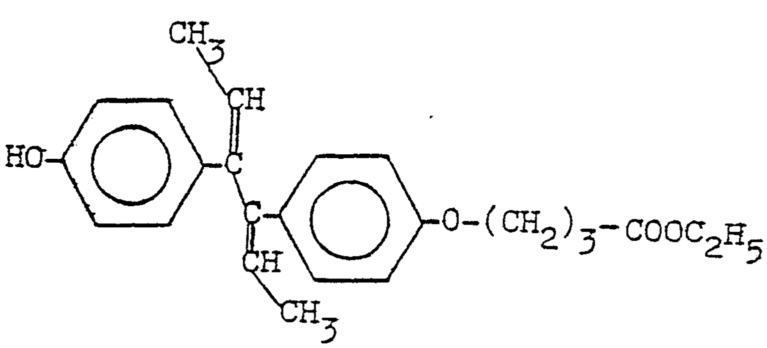

et l'hexestrol 4-0-monocarbéthoxypropyl de formule:

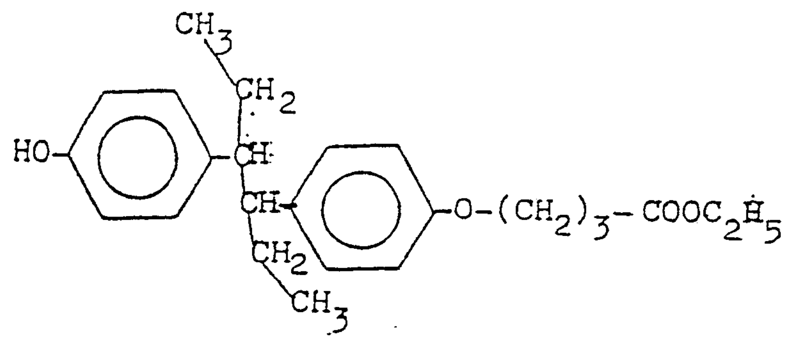

- le diénestrol 4-0-monocarboxypropyl de formule

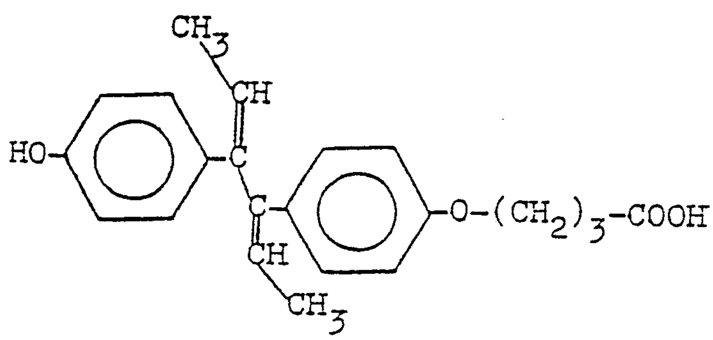

et l'hexestrol 4-0-monocarboxypropyl de formule

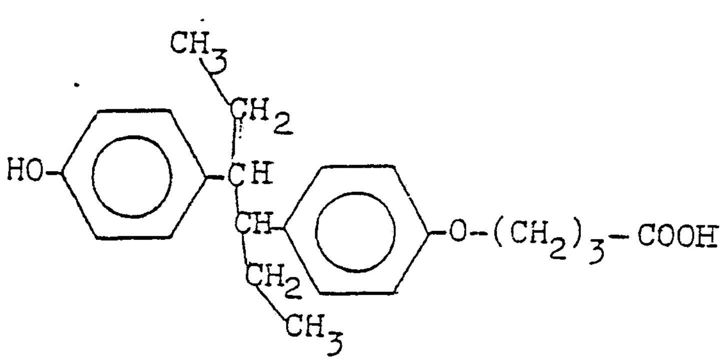

- l'anhydride mixte du diénestrol 4-0-monocarboxyprol avec le formiate d'isobutyle de formule

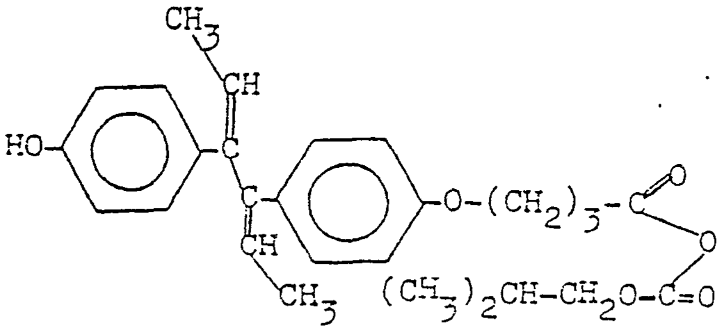

- l'anhydride mixte d'hexestrol 4-0-monocarboxypropyl avec le formiate d'isobutyle de formule:

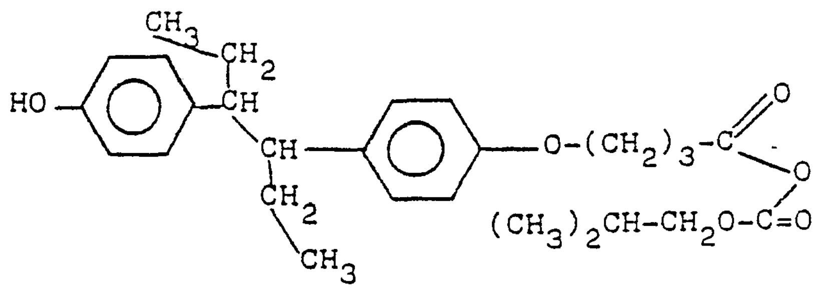

Les produits de formule générale VI, plus particulièrement, ceux répondant à la formule IV, sont des produits de départ utiles pour la préparation d'antigènes, nécessaires également aux dosages radioimmunologiques du diéthyl-stilbestrol, du diénestrol et d'hexestrol.

Les produits de formule générale I, objet de l'invention, et notamment le dienestrol 4-0-monocarboxypropyl ($^{125}I$) histamine et l'hexestrol 4-0-monocarboxypropyl ($^{125}I$) histamine sont utilisés comme déjà dit, lors du dosage radioimmunologique du diénestrol, de l'hexestrol, ainsi que du diéthylstilbestrol.

A l'appui de ceci, on peut citer, à titre d'exemple, l'analyse possible de traces de ces stilbènes dans les carcasses, tissus, liquides corporels, excretats de bovins.

Ils permettent aussi la dosage da ces stilbènes dans les liquides et tissus biologiques et dans l'alimentation

humaine et animale.

Le dosage radioimmunologique est effectué selon la méthode décrite par S.A. Bergson et R.S. Yalow Hormone 4 p. 557 (1964) et G.E. Abraham "Journal of Chemical endocrinonal metab" 29 p. 866 (1969).

Les exemples suivants illustrent l'invention.

**Exemple I: 4-0-monocarboxypropyl diénestrol couplé à l'($^{125}$I) histamine.**

**Stade A: Diénestrol 4-0-monoéthoxycarbonylpropyle**

On prépare un mélange de 20 g de diénestrol, 7 g de bromobutyrate d'éthyle et 4,6 g de carbonate de potassium sec dans 300 ml d'acétone, porte ce mélange au reflux pendant 17 heures environ, le laisse refroidir ensuite à température ambiante, le verse dans l'eau, extrait à l'acétate d'éthyle, lave les phases organiques réunies, à l'eau, les sèche, les traite au charbon actif, évapore le solvant et sèche sous pression réduite. On recueille 24,4 g d'un résidu semi cristallisé que l'on purifie par chromatographie sur silice sous pression avec elution par le mélange: chlorure de méthylène/ méthanol (95/5) et obtient 6,36 g de produit cherché, P.F. vers 86°C.

**Spectre IR (chloroforme)**

OH vers 3590 $cm^{-1}$
C = 0 vers 1728 $cm^{-1}$
1610 $cm^{-1}$
1590 $cm^{-1}$
1573 $cm^{-1}$
1510 $cm^{-1}$

**Stade B: Diénestrol 4-0-monocarboxypropyl**

On introduit dans 155 ml de méthanol 6,16 g de produit obtenu au stade précédent et 31 ml de soude 2N, porte la solution résultante au reflux pendant 1 heure Après refroidissement à température ambiante, on amène le pH à 5-6 par addition de 7 ml d'acide acétique, verse le mélange réactionnel dans l'eau, extrait à l'acétate d'éthyle, réunit les phases organiques, les lave à 1,eau, les sèche, traite au charbon actif, évapore le solvant concentre à sec sous pression réduite et obtient 5,48 g de produit brut. On purifie ce produit par le passage au sel de sodium et le retour à l'acide libre et obtient 5,16 g de produit cherché; P.F. = 158° C.

**Analyse**: $C_{22}H_{24}O_4$

| | C % | H % |
|---|---|---|
| Calculé | 74,98 | 6,86 |
| Trouvé | 74,7 | 7,0 |

**Stade C: Anhydride mixte de formiate d'isobutyle et diénestrol-4-0-monocarboxypropyl**

On mélange successivement 2,5 mg de produit préparé au stade précédent, 50 µl de dioxane, 10 µl d'un mélange dioxane-tri-n-butylamine (5: 1) et 10 µl de mélange dioxane -chloroformiate d'isobutyle (10: 1). La solution est agitée pendant 30 minutes sous atmosphère inerte, puis on ajoute 3,4 ml de dioxane. On obtient une solution du produit cherché que l'on utilise telle quelle pour le stade suivant de la synthèse.

**Stade D: Dienestrol 4-0-monocarboxypropyl couplé à l'($^{125}$I) histamine.**

Iodation de l'histamine:

On prépare 10 µl d'une solution d'histamine $10^{-4}$M dans une solution tampon de phosphate de sodium 0,5 M de pH 8, puis on ajoute successivement 1 mCi de iodure $^{125}$I de sodium (activité spécifique 2000 Ci/mmol) dans 5 µl d'eau distillée et 50µg de chloramine T dans 10 µl d'eau distillée. On agite le mélange réactionnel pendant 75

secondes, ajoute une solution de 300 µg de métabisulfite de sodium dissous dans 10 µl d'eau distillée, et obtient une solution aqueuse du produit cherché Rf = 0,1 (chromatographie sur silice, système de solvant méthanol-triéthylamine 98: 2)

Condensation:

On ajoute à cette solution 50 µl de la solution d'anhydride mixte obtenué au stade précédent, agite et laisse à l'obscurité pendant deux heures à une température proche de 4°C. On dilue ensuite le mélange réactionnel avec 0,3 ml d'une solution aqueuse de bicarbonate de sodium $10^{-1}$M, extrait par 1,5 ml de chlorure de méthylène, évapore la phase organique sous atmosphère inerte, reprend le résidu par 100 µl de chlorure de méthylène, chromatographie sur silice en éluant par le mélange chloroforme-méthanol (97: 3) et réunit les fractions contenant le produit. Le produit obtenu présante une activité totale de 100 µCi.

**Exemple II: Hexestrol 4-0-monocarboxypropyl couplé à l' ($^{125}$I) histamine.**

En procédant comme décrit dans l'exemple I et au départ de l'hexestrol on obtient:
- 6,7 g d'hexestrol 4-0-monoéthoxycarbonyl propyl. PF vers 110° C.

**Spectre IR** (chloroforme)

OH vers 3590 $cm^{-1}$
C = 0 vers 1725 $cm^{-1}$
Aromatique 1610 $cm^{-1}$
1594 $cm^{-1}$
1580 $cm^{-1}$
1508 $cm^{-1}$
- 4,5 g d'hexestrol 4-0-monocarboxypropyl.
PF vers 183° C

**Analyse** $C_{22}R_{28}O_4$

| | C % | H % |
|---|---|---|
| Calculé | 74,13 | 7,92 |
| Trouvé | 74,4 | 8,1 |

- l'anhydride mixte dé l'hexestrol 4-0-monocarboxypropyl avec le formiate d'isobutyle.
- de l'hexestrol 4-0-monocarboxypropyl couplé à l'($^{125}$I) histamine, présentant une activité totale de 100 µCi.

**Revendications:** pour les Etats contractants BE CH DE GB IT LI NL SE

1) Dérivés de stilbèné radioactifs marqués à l'iodé $^{125}$ ou $^{131}$, de formule générale I:

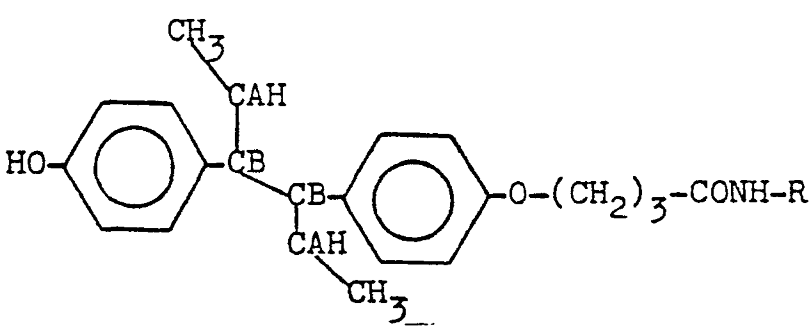

dans laquelle A et B représentent chacun un atome d'hydrogène ou réprésentent ensemble une double liaison et R représenté 1é reste d'un acidé aminé $RNH_2$ ou le reste d'un dérivé dé ce dernier, ce resté possédant un groupé accepteur d'iode et étant marqué à l'iode $^{125}$ ou $^{131}$.

2) Dérivés de stilbèné radioactifs marqués à l'iodé de formule générale I selon la révendication 1, dans laquelle R représente le reste d'un acide aminé $RNH_2$ ou le reste d'un dérivé de ce dernier, choisi dans le groupe constitué par l'histidine, la tyrosine, l'histamine, la tyraminé et le tyrosynate dé méthyle, marqués à l'iode $^{125}$ ou $^{131}$

3) Le diénestrol 4-0-monocarboxypropyle, couplé à l'($^{125}$I) histamine de formule:

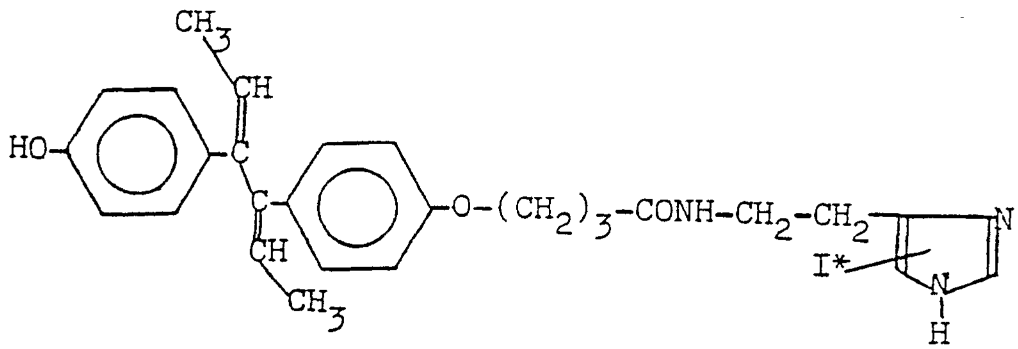

dans laquelle le signe * indique qu'il s'agit d'iode $^{125}$ radioactif, lequel iode $^{125}$ peut être en position 2 ou 5.

4) L'hexestrol 4-0-monocarboxypropyl couplé à 1' ($^{125}$I) histamine de formule:

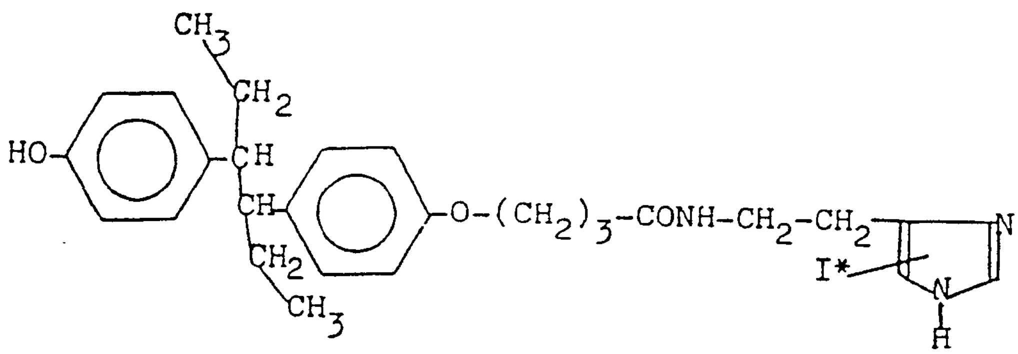

-dans laquelle le signe * indique qu'il s'agit d'iode[125] radioactif, lequel iode[125] peut être en position 2 ou 5.

5) Procédé de préparation des produits de formule I, tels que définis à la revendication 1, caractérisé en ce que:

- on fait réagir un produit de formule générale II:

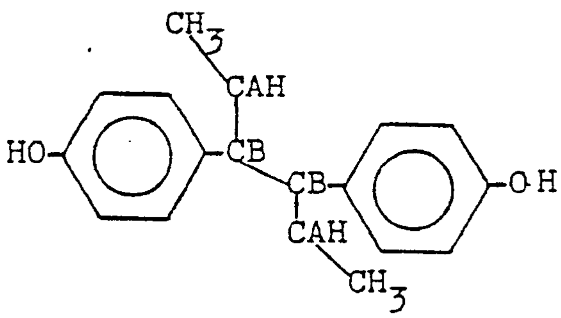

dans laquélle ét dans cé qui suit, A et B ont les significations précitées, avéc un halogéno butyrate d'alcoyle, en présénce d'une base, pour obtenir l'éther de formule générale III:

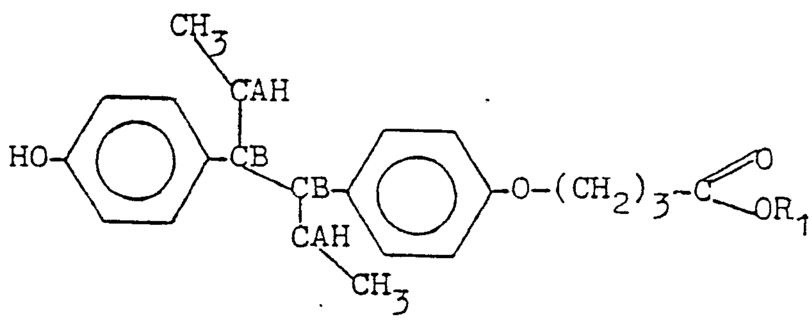

dans laquelle $R_1$ représente un radical alcoyle ayant au plus 6 atomes de carbone, dont on saponifie la fonction ester, pour obtenir un produit de formule généralé IV:

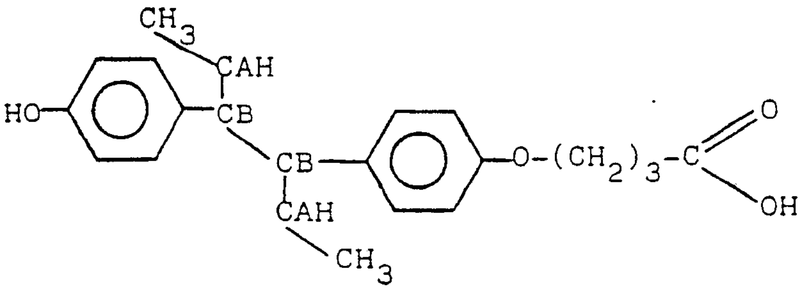

- puis fixe sur la fonction acide de ce produit de formule IV un groupement activateur de la fonction carbonyle et obtient le produit de formule générale V:

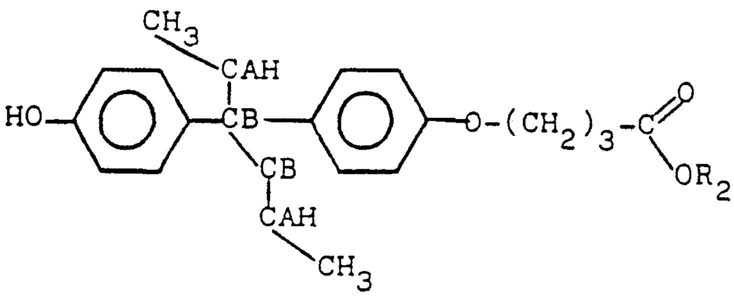

dans laquelle $R_2$ représente un groupement activateur de la fonction carbonylé,

- que l'on fait réagir avec un acide aminé accepteur d'iode ou avec un dérivé d'un tel acide et obtient lé produit cherché de formule générale I.

6) Procédé selon la revendication 5, caractérisé en cé que l'acide aminé ou le dérivé d'acidé aminé, accepteur d'iode, est choisi dans le groupé constitué par l'histidiné, la tyrosine l'histamine, la tyramine et le tyrosinate de méthyle.

7) Procédè selon la révendication 5 ou 6, caractérisé en ce qué:

l'on fixé sur la fonction acide du produit de formule générale IV, un groupement activateur de la fonction carbonyle en faisant réagir le produit de formulé IV avec un halogénoformiate d'alcoyle, en présence d'une base tértiaire, en milieu anhydre et sous atmosphère inerté et obtiént lé produit dé formulé générale V:

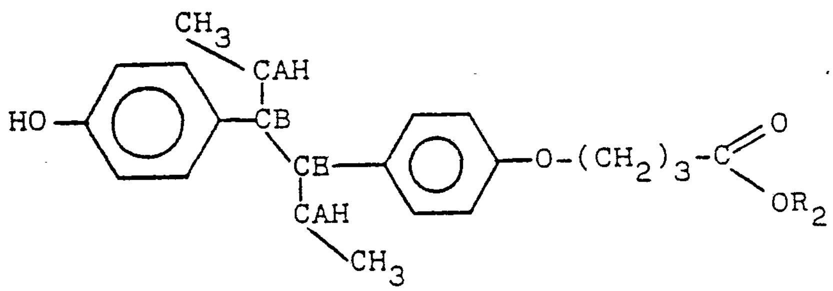

dans laquelle $R_2$ est un groupement activateur de la fonction carbonyle, de formule: -CO-O-Alk, Alk étant un radical alcoyle ayant au plus 6 atomes de carbone.

8) Procédé suivant l'une quelconque des revendications 5 à 7, caractérisé en ce que:

- l'halogénobutyrate d'alcoyle que l'on fait réagir sur le produit de formule générale II est le bromobutyrate d'éthyle et on opère en présence de carbonate de potassium;
- l'on saponifie la fonction ester du composé de formule generale III, au moyen de la soude methanolique;
- le produit permettant de fixer un groupement activateur de la fonction carbonyle que l'on fait réagir avec le produit de formule générale IV, est le chloroformiate d'isobutyle et l'on opère en présence de tri-n-butylamine, dans le dioxane anhydre et sous atmosphère inerte.

9) Procédé suivant l'une quelconque des revendications 5 à 8, caractérisé en ce que l'acide aminé ou son dérivé que l'on fait réagir avec le produit de formule générale V est l'histamine marquée à l'iode $^{125}$ ou $^{131}$, et que l'on opère sous atmosphère inerte.

10) Application des produits tels que décrits à l'une quelconque des revendications 1 à 4 aux dosagés radioimmunologiques du diénestrol, de l'hexestrol ainsi que du diéthylstilbestrol, dans les liquides et les tissus biologiques de l'homme ou de l'animal et dans l'alimentation humaine et animale.

11) A titre de produits industriels nouveaux, les produits de formule générale VI:

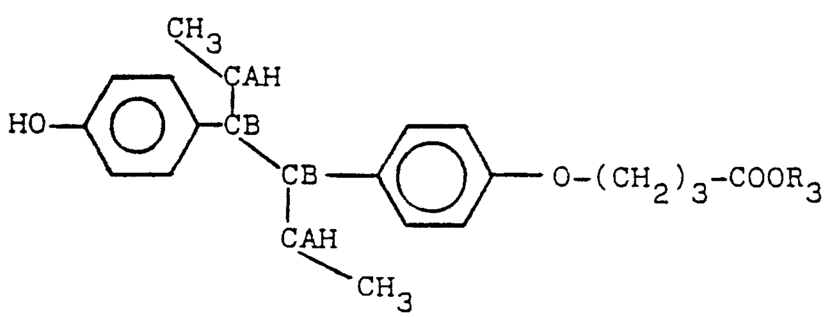

dans laquelle A et B représentent chacun un atome d'hydrogène ou représentant ensemble une double liaison et $R_3$ représente un atome d'hydrogène, un radical alcoyle ayant au plus 6 atomes de carbone ou un groupement activateur de la fonction carbonyle, qui est choisi dans le groupe constitué par le groupement -CO-O-Alk, Alk étant un radical alcoyle ayant au plus 6 atomes de carbone, ou les groupements résultant de l'action du N-hydroxy succinimide ou du dicyclohexylcarbodiimide sur la fonction carbonyle.

12) Produits selon la revendication 11, à savoir:

- le diénestrol 4-0-monocarbéthoxypropyle de formule:

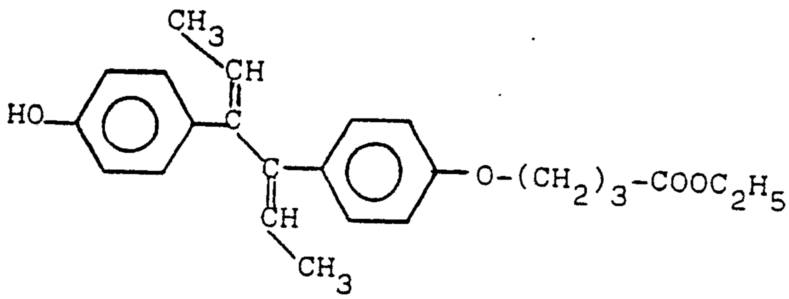

et l'hexestrol 4-0-monocarbéthoxypropyle de formule:

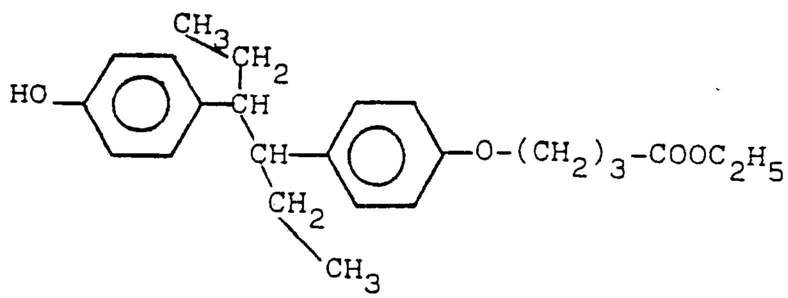

13) Produits selon la revendication 11, à savoir:
- le diénestrol 4-0-monocarboxypropyle de formule:

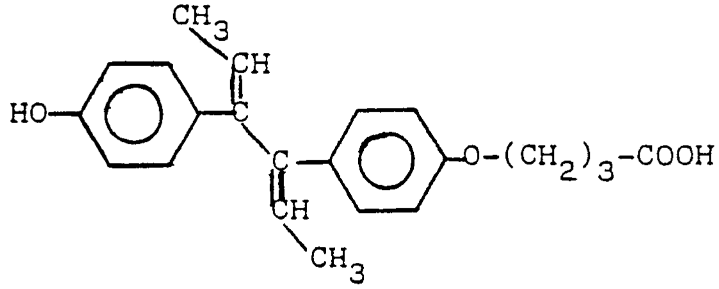

et l'hexestrol 4-0-monocarboxypropyle de formule:

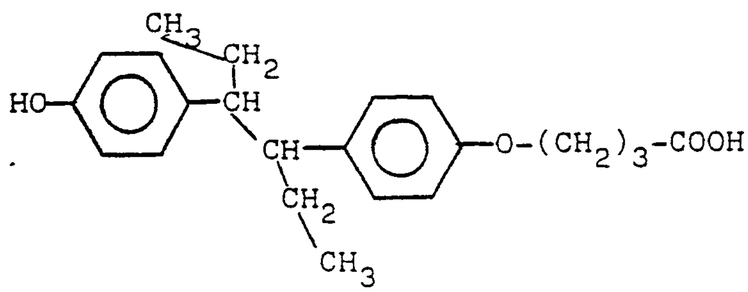

14) Produits selon la revendication 11, à savoir:
- l'anhydride mixte du diénestrol 4-0-monocarboxypropyl avec le formiate d'isobutyle de formule:

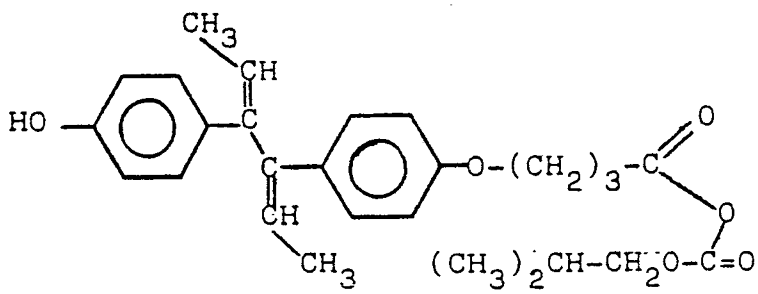

- l'anhydride mixte d'hexestrol 4-0-monocarboxypropyle avec le formiate d'isobutyle de formule:

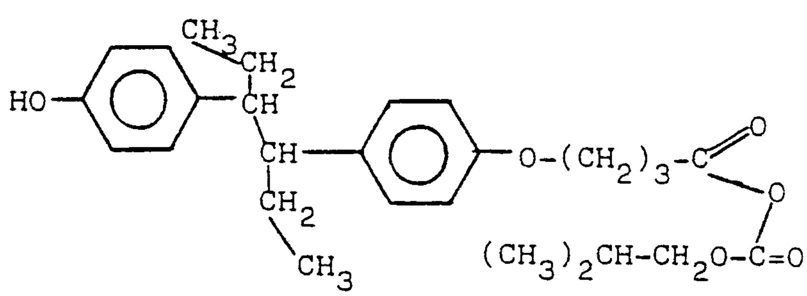

**Revendications:** pour l'Etat contractant AT

1) Procédé pour préparer les dérivés de stilbène radioactifs marqués à l'iode 125 ou 131, de formule générale I:

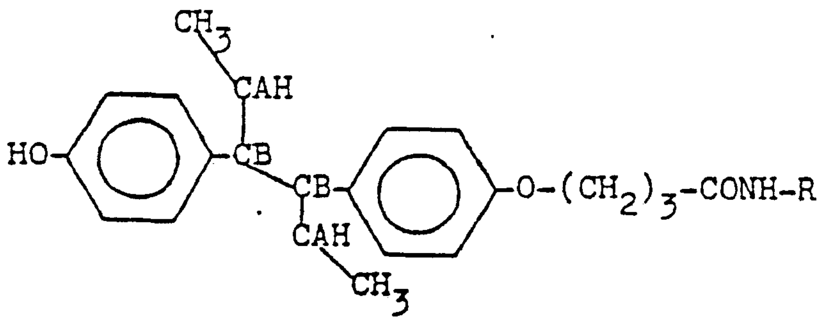

dans laquelle A et B représentent chacun un atome d'hydrogène ou représentent ensemble une double liaison et R représente le reste d'un acide aminé $RNH_2$ ou le reste d'un dérivé de ce dernier, ce reste possédant un groupe accepteur d'iode et étant marqués à l'iode 125 ou 131, caractérisé en ce qué:

- on fait réagir un produit de formule générale II:

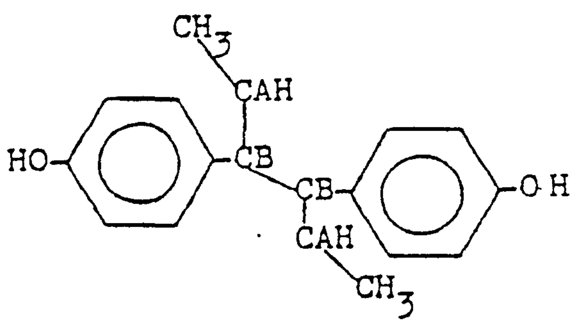

dans laquelle et dans ce qui suit, A et B ont les significations précitées, avec un halogéno butyrate d'alcoyle, en présence d'une base, pour obtenir l'éther de formule générale III:

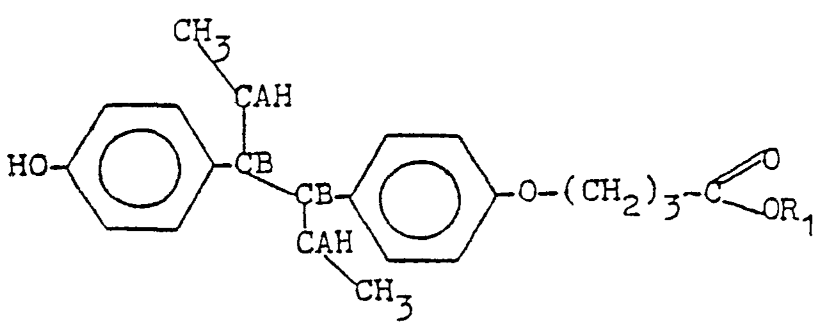

dans laquelle $R_1$ représente un radical alcoyle ayant au plus 6 atomes de carbone, dont on saponifie la fonction ester, pour obtenir un produit de formule générale IV:

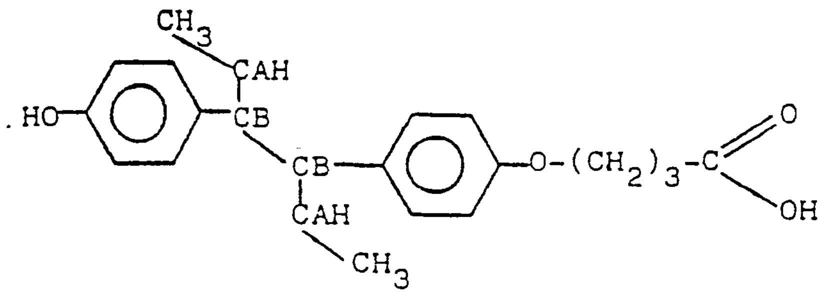

- puis fixé sur la fonction acide de ce produit de formule IV un groupement activateur de la fonction carbonyle et obtient le produit de formule générale V:

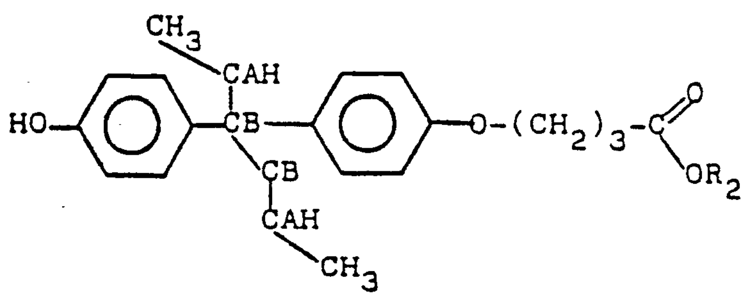

dans laquelle $R_2$ représente un groupement activateur de la fonction carbonyle,

- que l'on fait réagir avec un acide aminé accepteur d'iode ou avec un dérivé d'un tel acidé et obtient le produit cherché de formule générale I.

2) Procédé selon la revendication 1, caractérisé en ce que l'acide aminé ou le dérivé d'acide aminé, accepteur d'iode, est choisi dans le groupe constitué par l'histidiné, la tyrosine, l'histamine, la tyramine et le tyrosinate de méthyle.

3) Procédé selon la revendication 1 ou 2, caractérisé en ce que l'on fixé sur la fonction acide du produit de formule générale IV, un groupement activateur de la fonction carbonyle, en faisant réagir le produit de formule IV avec un halogénoformiate d'alcoyle, en présence d'une base tertiaire, en milieu anhydre et sous atmosphère inerte et obtient le produit de formule générale V:

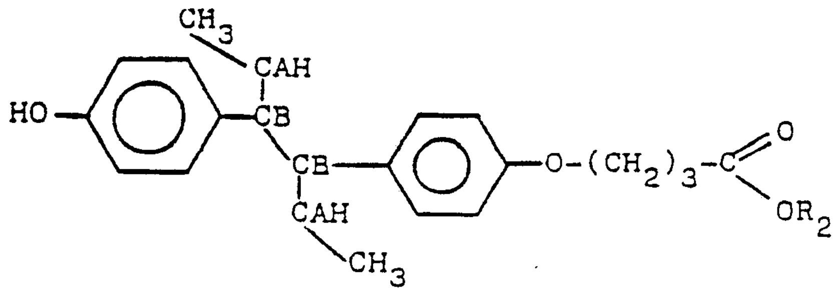

dans laquelle $R_2$ est un groupement activateur de la fonction carbonyle, de formule: -CO-O-Alk, Alk étant un radical alcoyle ayant au plus 6 atomes de carbone.

4) Procédé suivant l'une quelconque des revendications 1 à 3, caractérisé en ce que:

- l'halogénobutyrate d'alcoyle que l'on fait réagir sur le produit de formule générale II est le bromobutyrate d'éthyle et on opère en présence de carbonate de potassium;
- l'on saponifie la fonction ester du composé de formule générale III au moyen de la soude méthanolique.

5) Procédé suivant l'une quelconque des revendications 1 à 4, caractérisé en ce que l'acide aminé ou son dérivé que l'on fait réagir avec le produit de formule générale V est l'histamine marquée à l'iode 125 ou 131, et que l'on opère sous atmosphère inerte.

6) Procédé suivant l'une quelconque des revendications 1 à 5, caractérisé en ce que l'on utilise au départ, le diénestrol et que l'on obtient le diénestrol 4-0-monocarboxy-propyle couplé à l'($^{125}$I)-histamine de formule:

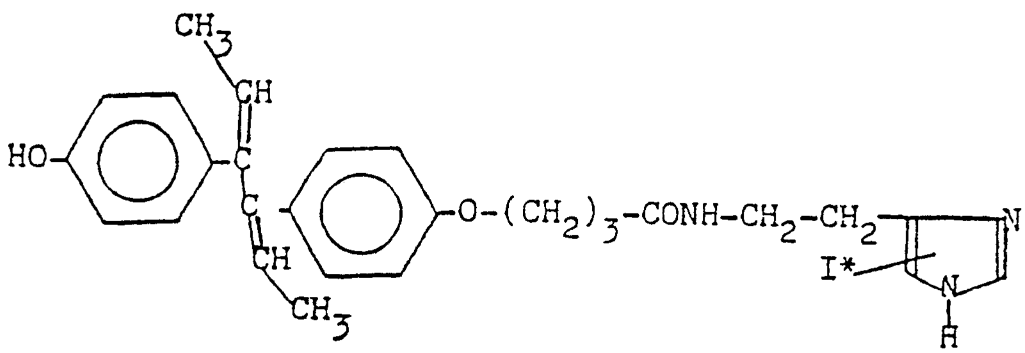

dans laquelle le signe * indique qu'il s'agit d'iode 125 radioactif, lequel iode 125 peut être en position 2 ou 5.

7) Procédé suivant l'une quelconque des revendications 1 à 5, caractérisé en ce que l'on utilise au départ l'hexestrol et qué l'on obtient l'hexestrol 4-0-monocarboxypropyle couplé à l'($^{125}$I)-histamine de formule:

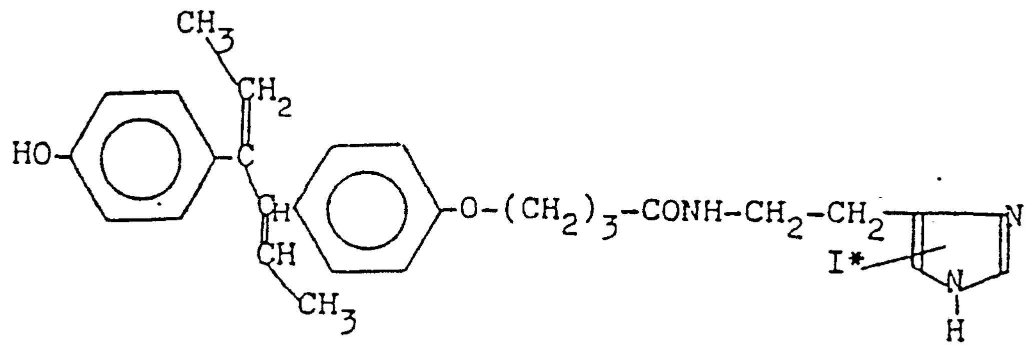

dans laquelle le signe * indique qu'il s'agit d'iode 125 radioactif, lequel iode125 peut être en position 2 ou 5. 8) Application des produits tels que décrits à l'une quelconque des revendications 1 et 5 à 7 aux dosages radioimmunoloques du diénestrol, de l'hexestrol ainsi que du diéthylstilbestrol dans les liquides et les tissus biologiques de l'homme ou de l'animal et dans l'aliméntation humaine et animale.

**Patentansprüche** für die Vertragsstaaten: BE, CH, DE, GB, IT, LI, NL, SE

1. $Jod^{125}$- oder $Jod^{131}$-markierte, radioaktive Stilbenderivate der allgemeinen Formel I

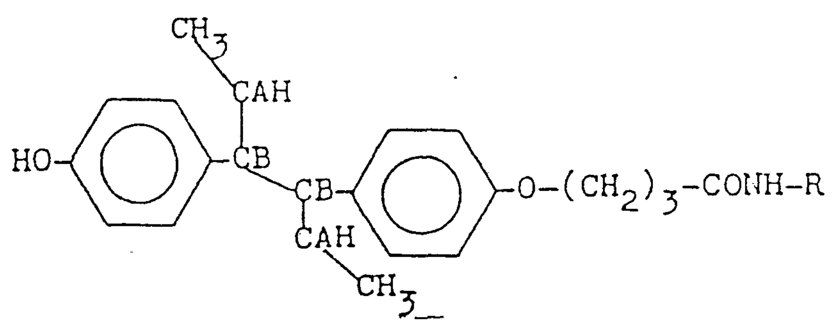

worin A und B jeweils ein Wasserstoffatom darstellen oder gemeinsam eine Doppelbindung bedeuten und R den Rest einer Aminosäure $RNH_2$ oder den Rest eines Derivats dieser letzteren darstellt, wobei dieser Rest eine Jodakzeptorgruppe besitzt und mit $Jod^{125}$ oder $J^{131}$ markiert ist.

2. Jodmarkierte, radioaktive Stilbenderivate der allgemeinen Formel I gemäß Anspruch 1, worin R den Rest einer Aminosäure $RNH_2$ oder den Rest eines Derivats dieser letzteren, ausgewählt unter Histidin, Tyrosin, Histamin, Tyramin und Methyltyrosinat, markiert mit $Jod^{125}$ oder $Jod^{131}$, bedeutet.

3. Mit ($^{125}J$)-Histamin gekuppeltes 4-0-Monocarboxypropyl-dienestrol der Formel

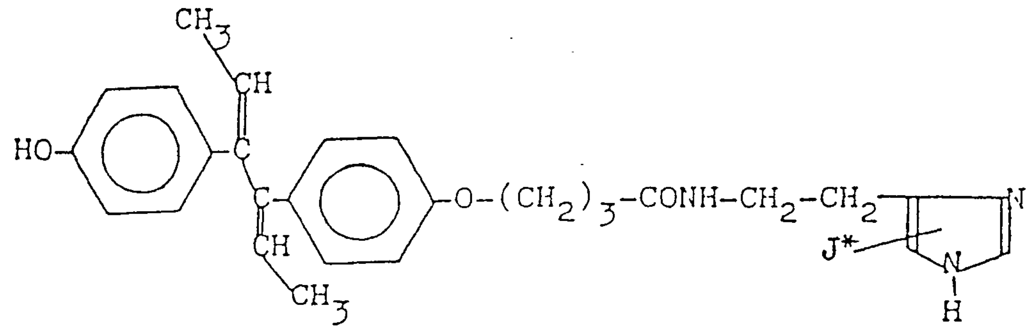

worin das Zeichen * angibt, daß es sich um radioaktives $Jod^{125}$ handelt, wobei sich das $Jod^{125}$ in 2- oder 5-Stellung befinden kann.

4. Mit ($^{125}J$)-Histamin gekuppeltes 4-0-Monocarboxypropyl-hexestrol der Formel

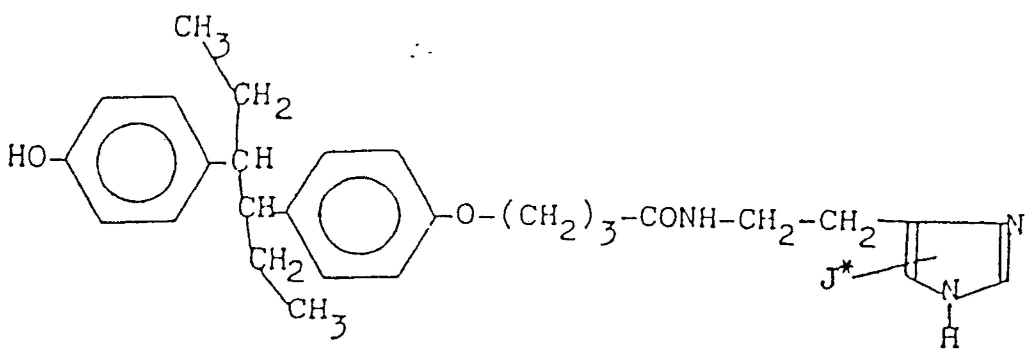

worin das Zeichen * angibt, daß es sich um radioaktives $Jod^{125}$ handelt, wobei sich das $Jod^{125}$ in 2- oder 5-Stellung befinden kann.

5. Verfahren zur Herstellung eines Produktes der Formel I gemäß Anspruch 1,
dadurch gekennzeichnet, daß
man ein Produkt der allgemeinen Formel II

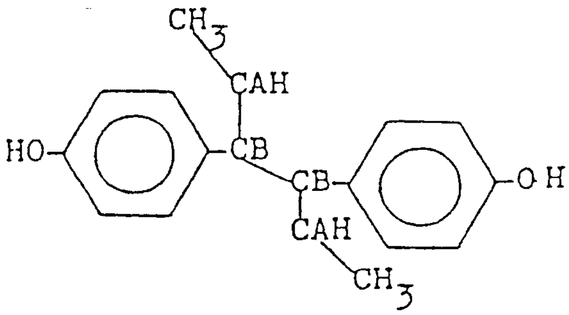

in der und im folgenden A und B die vorstehenden Definitionen besitzen, mit einem Alkylhalogenobutyrat in Anwesenheit einer Base umsetzt, um den Ether der allgemeinen Formel III

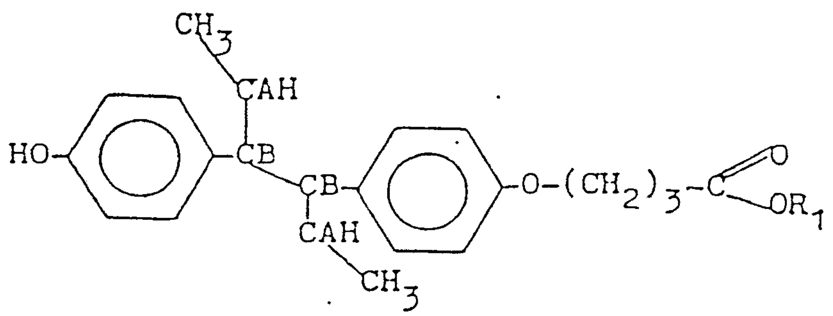

zu erhalten, worin $R_1$ einen Alkylrest mit höchstens 6 Kohlenstoffatomen bedeutet, dessen Esterfunktion man verseift, um ein Produkt der allgemeinen Formel IV

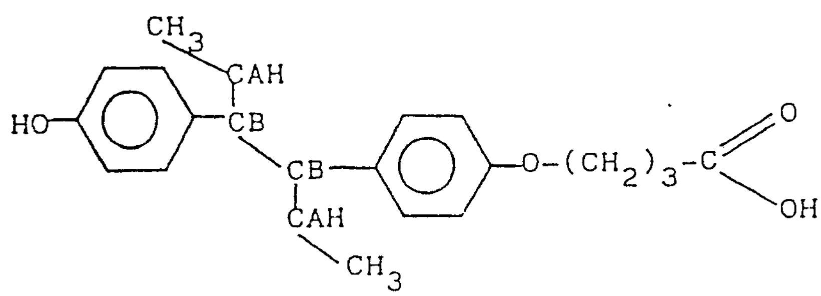

zu erhalten,

anschließend an der Säurefunktion dieses Produktes der Formel IV eine Aktivatorgruppe der Carbonylfunktion fixiert und das Produkt der allgemeinen Formel V

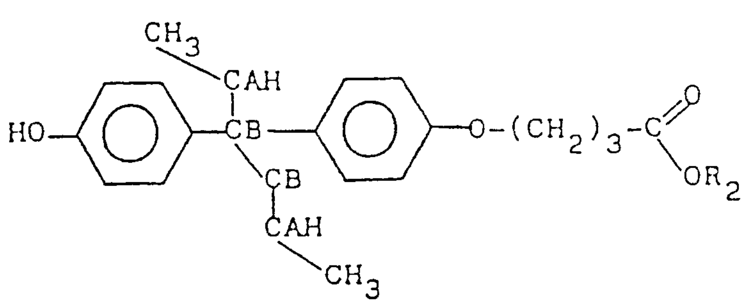

erhält, worin $R_2$ eine Aktivatorgruppe der Carbonylfunktion bedeutet,

welches man mit einer Jodakzeptor-Aminosäure oder mit einem Derivat einer derartigen Säure umsetzt und das gewünschte Produkt der allgemeinen Formel I erhält.

6. Verfahren gemäß Anspruch 5, dadurch gekennzeichnet, daß die Aminosäure und das Derivat dieser Aminosäure, der Jodakzeptor, unter Histidin, Tyrosin, Histamin, Tyramin und Methyltyrosinat ausgewählt werden.

7. Verfahren gemäß Anspruch 5 oder 6, dadurch gekennzeichnet, daß man an der Säurefunktion des Produkts der allgemeinen Formel IV eine Aktivatorgruppe der Carbonylfunktion fixiert, indem man das Produkt der Formel IV mit einem Alkylhalogenoformiat in Anwesenheit einer tertiären Base in wasserfreiem Milieu und unter inerter Atmosphäre umsetzt und das Produkt der allgemeinen Formel V

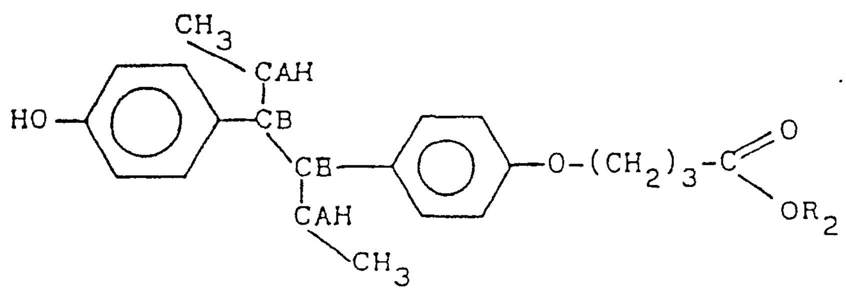

erhält, worin $R_2$ eine Aktivatorgruppe der Carbonylfunktion der Formel -CO-O-Alk ist, wobei Alk einen Alkylrest mit höchstens 6 Kohlenstoffatomen darstellt.

8. Verfahren gemäß einem der Ansprüche 5 bis 7, dadurch gekennzeichnet, daß das Alkylhalogenobutyrat, das man mit dem Produkt der allgemeinen Formel II umsetzt, Ethylbromobutyrat ist, und man in Anwesenheit von Kaliumcarbonat arbeitet;
man die Esterfunktion der Verbindung der allgemeinen Formel III mit methanolischer Natronlauge verseift;
das die Fixierung einer Aktivatorgruppe der Carbonylfunktion ermöglichende Produkt, das man mit dem Produkt der allgemeinen Formel IV umsetzt, Isobutylchloroformiat ist und man in Anwesenheit von Tri-n-butylamin in wasserfreiem Dioxan und unter inerter Atmosphäre arbeitet.

9. Verfahren gemäß einem der Ansprüche 5 bis 8, dadurch gekennzeichnet, daß die Aminosäure oder ihr Derivat, welche man mit dem Produkt der allgemeinen Formel V umsetzt, $Jod^{125}$- oder $Jod^{131}$-markiertes Histamin ist und daß man unter inerter Atmosphäre arbeitet.

10. Verwendung der Produkte gemäß einem der Ansprüche 1 bis 4 für radioimmunologische Bestimmungen von Dienestrol, Hexestrol sowie Diethylstilbestrol in biologischen Flüssigkeiten und Geweben von Mensch oder Tier und in der menschlichen und tierischen Nahrung.

11. Als neue industrielle Produkte die Produkte der allgemeinen Formel VI

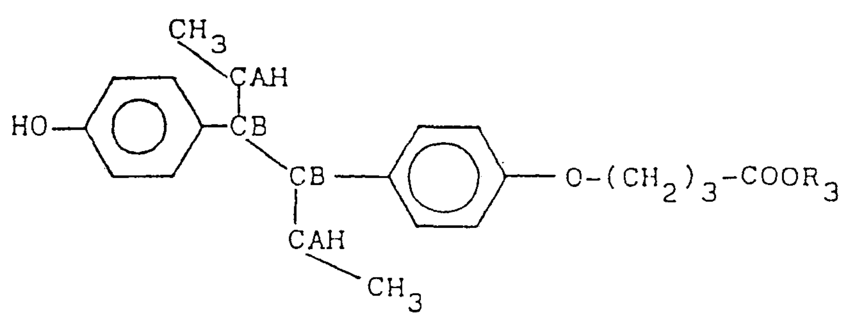

worin A und B jeweils ein Wasserstoffatom bedeuten oder gemeinsam eine Doppelbindung darstellen und $R_3$ ein Wasserstoffatom, einen Alkylrest mit höchstens 6 Kohlenstoffatomen oder eine Aktivatorgruppe der Carbonylfunktion, die ausgewählt wird unter -CO-O-Alk, wobei Alk einen Alkylrest mit höchstens 6 Kohlenstoffatomen bedeutet, oder den Gruppen, die der Umsetzung von N-Hydroxysuccinimid oder Dicyclohexylcarbodiimid mit der Carbonylfunktion entstammen, bedeutet.

12. Produkte gemäß Anspruch 11, nämlich 4-0-Monocarbethoxypropyl-dienestrol der Formel

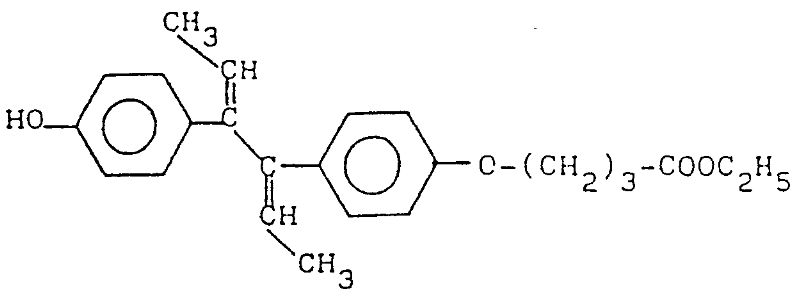

und 4-0-Monocarbethoxypropyl-hexestrol der Formel

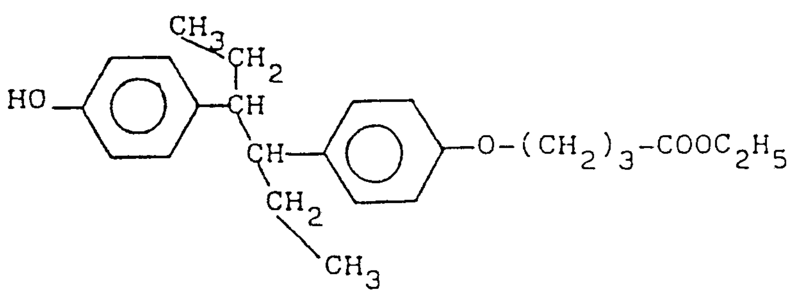

13. Produkte gemäß Anspruch 11, nämlich 4-0-Monocarboxypropyl-dienestrol der Formel

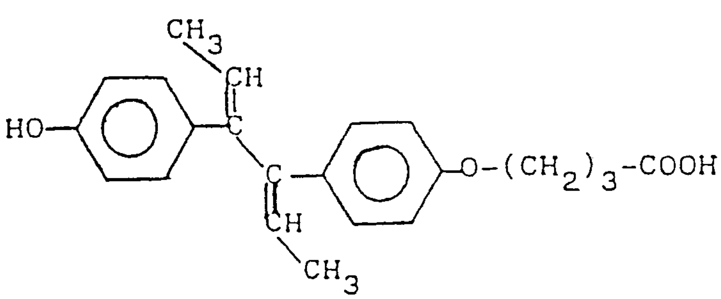

und 4-0-Monocarboxypropyl-hexestrol der Formel

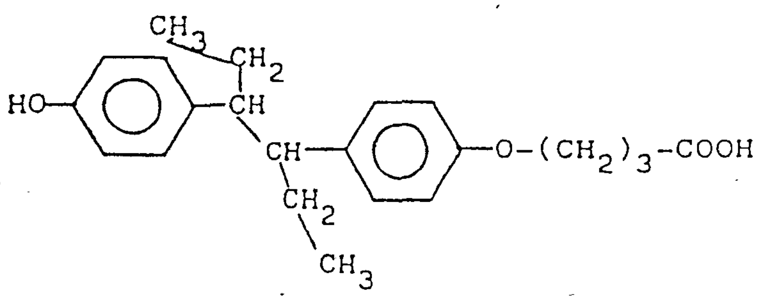

14. Produkte gemäß Anspruch 11, nämlich das gemischte Anhydrid von 4-0-Monocarboxypropyl-dienestrol mit Isobutylformiat der Formel

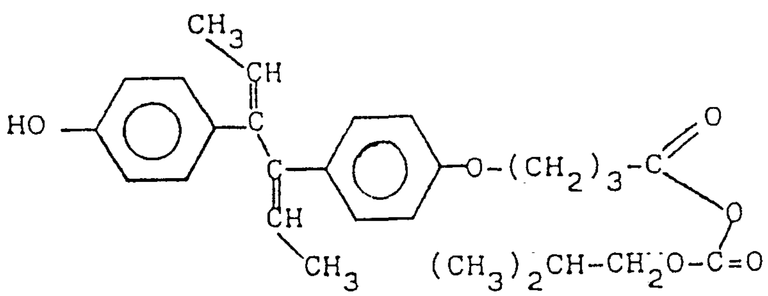

das gemischte Anhydrid von 4-0-Monocarboxypropyl-hexestrol mit Isobutylformiat der Formel

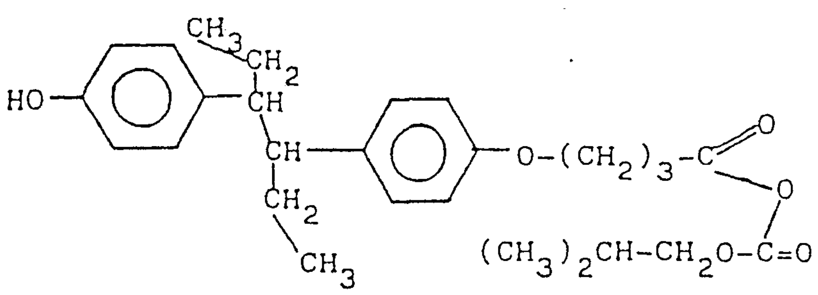

**Patentansprüche** für den Vertragsstaat: AT

1. Verfahren zur Herstellung von radioaktiven, mit $Jod^{125}$ oder $Jod^{131}$ markierten stilbenderivaten der allgemeinen Formel I

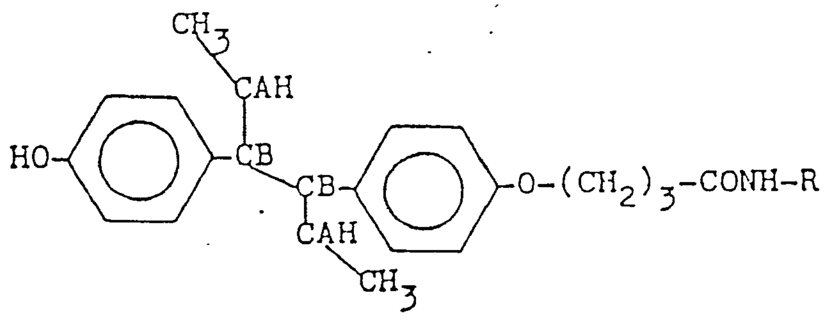

worin A und B jeweils ein Wasserstoffatom bedeuten oder gemeinsam eine Doppelbindung darstellen und R den Rest einer Aminosäure $RNH_2$ oder den Rest eines Derivats dieser letzteren bedeutet, wobei dieser Rest eine Jodakzeptorgruppe besitzt und mit $Jod^{125}$ oder $Jod^{131}$ markiert ist,

dadurch gekennzeichnet, daß

man ein Produkt der allgemeinen Formel II

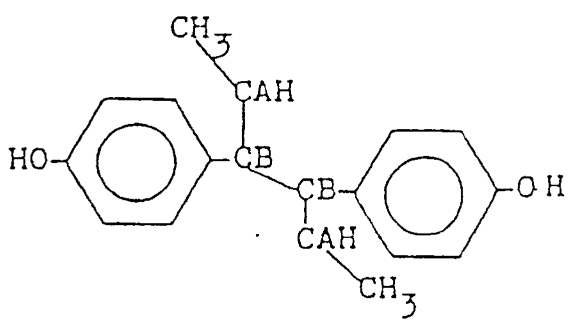

in der und im folgenden A und B die vorstehenden Definitionen besitzen, mit einem Alkylhalogenobutyrat in Anwesenheit einer Base umsetzt, um den Ether der allgemeinen Formel III

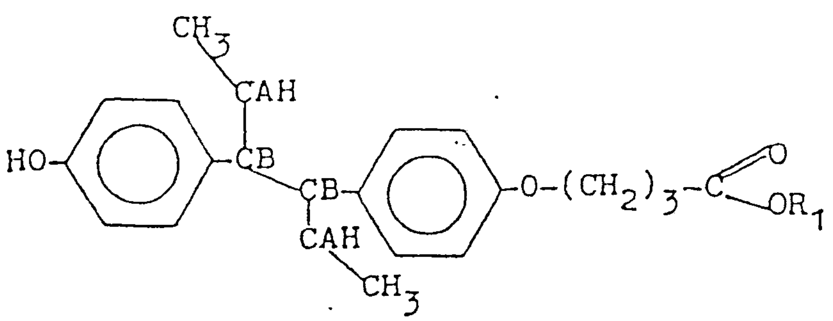

zu erhalten, worin $R_1$ einen Alkylrest mit höchstens 6 Kohlenstoffatomen bedeutet, dessen Esterfunktion man verseift, um ein Produkt der allgemeinen Formel IV

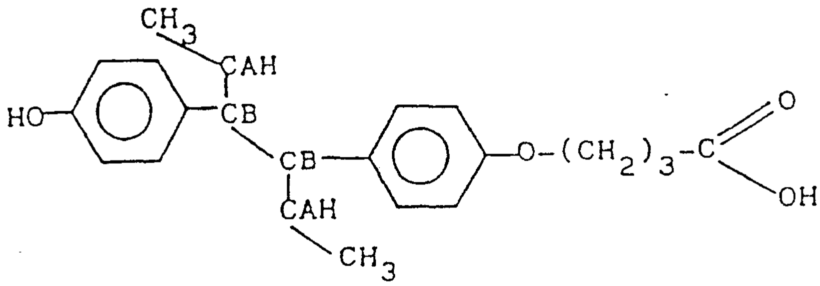

zu erhalten,

anschließend an der Säurefunktion dieses Produktes der Formel IV eine Aktivatorgruppe der Carbonylfunktion fixiert und das Produkt der allgemeinen Formel V

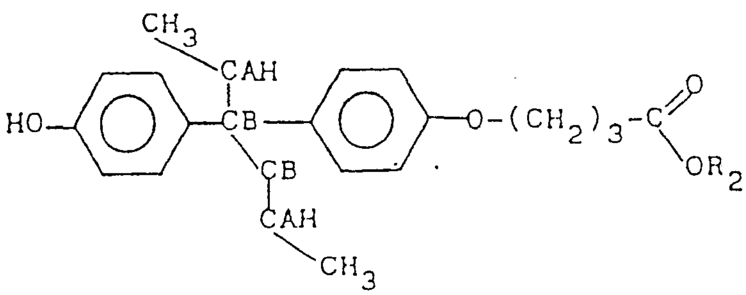

erhält, worin $R_2$ eine Aktivatorgruppe der Carbonylfunktion bedeutet,

welches man mit einer Jodakzeptor-Aminosäure oder mit einem Derivat einer derartigen 8äure umsetzt und das gewünschte Produkt der allgemeinen Formel I erhält.

2. Verfahren gemäß Anspruch 1, dadurch gekennzeichnet, daß die Aminosäure oder das Derivat der Aminosäure, der Jodakzeptor, unter Histidin, Tyrosin, Histamin, Tyramin und Methyltyrosinat ausgewählt wird.

3. Verfahren gemäß Anspruch 1 oder 2, dadurch gekennzeichnet, daß

man an der Säurefunktion des Produkts der allgemeinen Formel IV eine Aktivatorgruppe der Carbonylfunktion fixiert, indem man das Produkt der Formel IV mit einem Alkylhalogenoformiat in Anwesenheit einer tertiären Base in wasserfreiem Milieu und unter inerter Atmosphäre umsetzt und das Produkt der allgemeinen Formel V

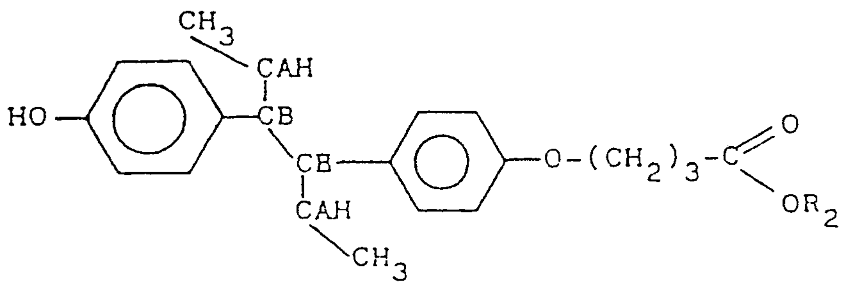

erhält, worin $R_2$ eine Aktivatorgruppe der Carbonylfunktion der Formel -CO-O-Alk ist, wobei Alk einen Alkylrest mit höchstens 6 Kohlenstoffatomen darstellt.

4. Verfahren gemäß einem der Ansprüche 1 bis 3, dadurch gekennzeichnet, daß

das Alkylhalogenobutyrat, das man mit dem Produkt der allgemeinen Formel II umsetzt, Ethylbromobutyrat ist, und man in Anwesenheit von Kaliumcarbonat arbeitet;

man die Esterfunktion der Verbindung der allgemeinen Formel III mit methanolischer Natronlauge verseift.

5. Verfahren gemäß einem der Ansprüche 1 bis 4, dadurch gekennzeichnet, daß die Aminosäure oder ihr Derivat, welche man mit dem Produkt der allgemeinen Formel V umsetzt, mit Jod$^{125}$ oder Jod$^{131}$ markiertes Histamin ist und man unter inerter Atmosphäre arbeitet.

6. Verfahren gemäß einem der Ansprüche 1 bis 5, dadurch gekennzeichnet, daß man von Dienestrol ausgeht und man mit ($^{125}$J)-Histamin gekuppeltes 4-0-Monocarboxypropyl-dienestrol der Formel

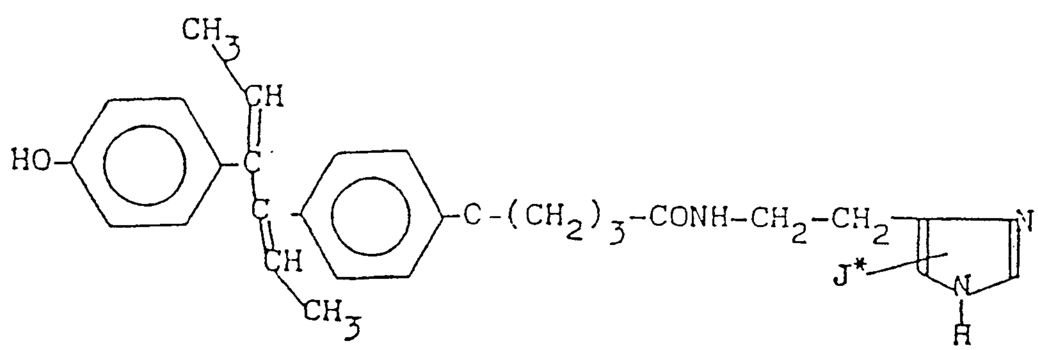

erhält, worin das Zeichen * angibt, daß es sich um radioaktives Jod$^{125}$ handelt, wobei sich das Jod$^{125}$ in 2- oder 5-Stellung befinden kann.

7. Verfahren gemäß einem der Ansprüche 1 bis 5, dadurch gekennzeichnet, daß man von Hexestrol ausgeht und mit($^{125}$J)-Histamin gekuppeltes 4-0-Monocarboxypropyl-hexestrol der Formel

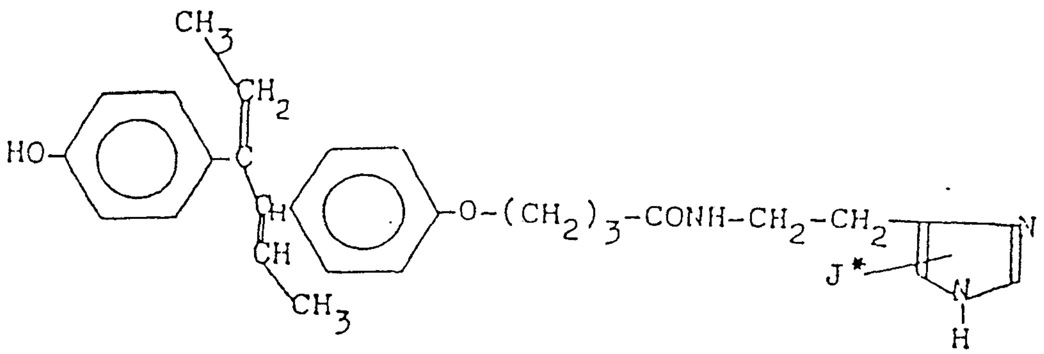

erhält, worin das Zeichen * angibt, daß es sich um radioaktives Jod[125] handelt, wobei sich das Jod[125] in 2- oder 5-Stellung befinden kann.

8. Verwendung der Produkte gemäß einem der Ansprüche 1 und 5 bis 7 für radioimmunologische Bestimmungen von Dienestrol, Hexestrol sowie Diethylstilbestrol in biologischen Flüssigkeiten und Geweben von Mensch oder Tier und in der menschlichen und tierischen Nahrung.

**Claims:** for the contracting states BE,CH,DE,GB,IT,LI,NL,SE.

1) Derivatives of radioactive stilbene marked with iodine[125] or [131], with the general formula:

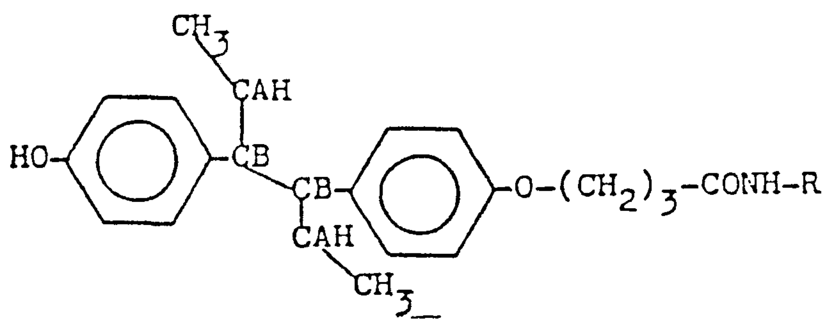

in which A and B each represent a hydrogen atom or together represent a double bond and R represents the residue of an amino acid $RNH_2$ or the residue of a derivative of this latter, this residue possessing an iodine acceptor group and being marked with iodine [125] or [131].

2) Derivatives of radioactive stilbene marked with iodine, with the general formula I, according to Claim 1, in which R represents the residue of an amino acid $RNH_2$ or the residue of a derivative of this latter, chosen from the group constituted by histidine, tyrosine, histamine, tyramine and methyl tyrosynate, marked with iodine [125] or [131].

3) 4-0-monocarboxypropyl dienestrol, coupled to ($^{125}$I) histamine with the formula:

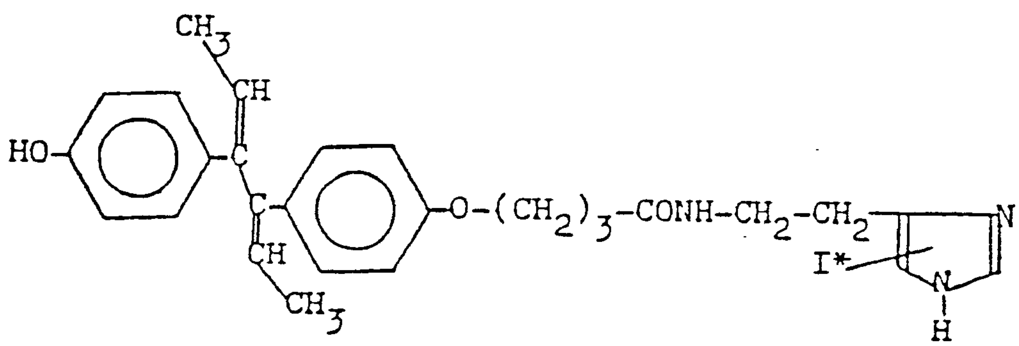

in which the sign * indicates that it concerns radioactive iodine $^{125}$, which iodine $^{125}$ can be in position 2 or 5.

4) 4-0-monocarboxypropyl hexestrol, coupled to ($^{125}$I) histamine with the formula:

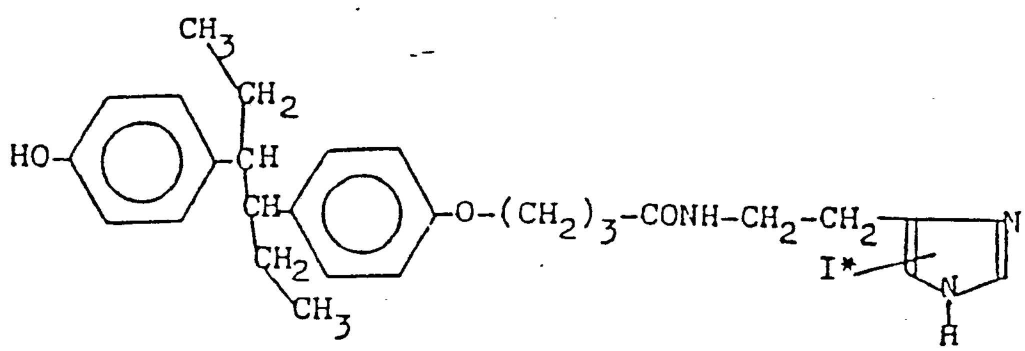

in which the sign * indicates that radioactive iodine $^{125}$ is concerned, which iodine$^{125}$ can be in position 2 or 5.

5) Process for the preparation of the products with the formula I, as defined in Claim 1, characterized in that - a product with the general formula II:

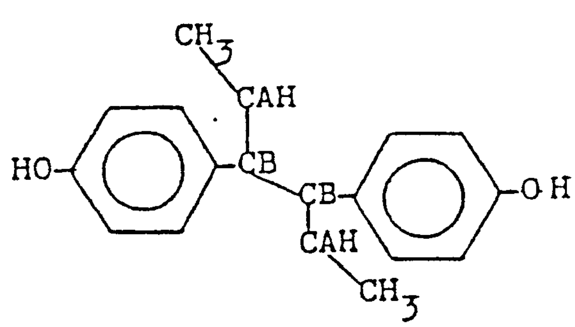

in which, and in what follows, A and B have the previously stated significances, is made to react with an alkyl halogeno butyrate, in the presence of a base, so as to obtain the ether with the general formula III:

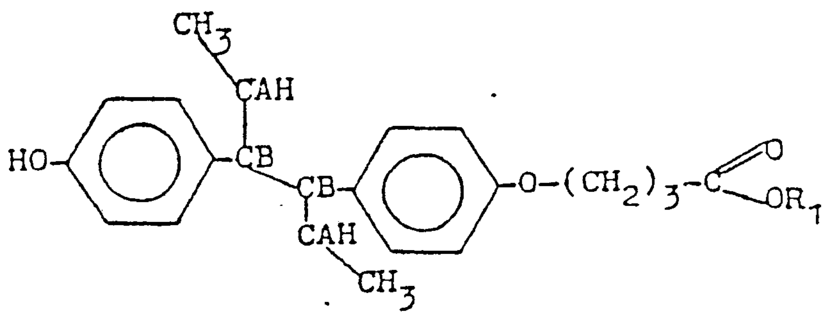

III

in which $R_1$ represents an alkyl radical having at the most 6 carbon atoms, of which the ester function is saponified, so as to obtain a product with the general formula IV:

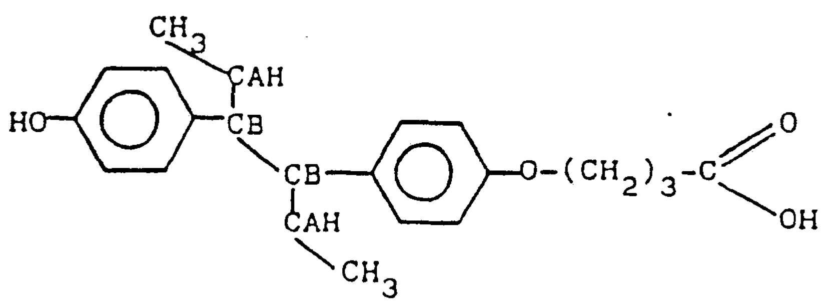

- then on the acid function of this product with the formula IV an activator group for the carbonyl function is fixed, and there is obtained the product with the general formula V:

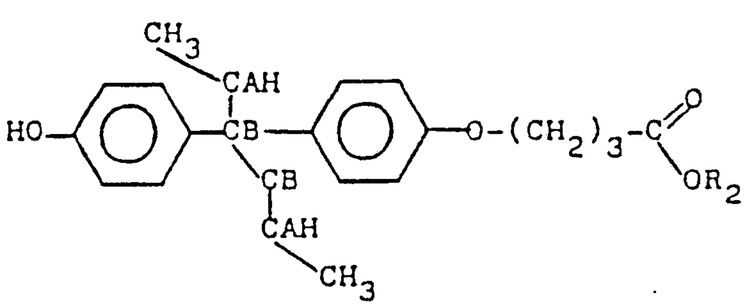

in which $R_2$ represents an activator group for the carbonyl function,

- which is made to react with an amino acid iodine acceptor or with a derivative of such an acid, and the product sought with the general formula I is obtained.

6) Process according to Claim 5, characterized in that the amino acid or the derivative of the amino acid, iodine acceptor is chosen from the group constituted by histidine, tyrosine, histamine, tyramine and methyl tyrosinate.

7) Process according to Claim 5 or 6, characterized in that: an activator group for the carbonyl function is fixed

on the acid function of the product with the general formula IV by making the product with the formula IV react with an alkyl halogenoformate in the presence of a tertiary base, in an anhydrous medium and in an inert atmosphere, so obtaining the product with the general formula V:

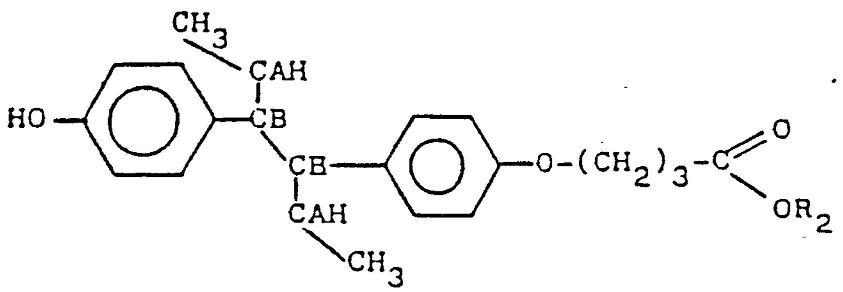

in which $R_2$ is an activator group for the carbonyl function, with the formula: -CO-O-Alk, Alk being an alkyl radical having at the most 6 carbon atoms.

8) Process according to any one of the claims 5 to 7, characterized in that

- the alkyl halogenobutyrate which is made to react on the product with the general formula II is ethyl bromobutyrate and the operation is done in the presence of potassium carbonate
- the ester function of the compound with the general formula III is saponified by means of sodium hydroxide in methanol
- the product enabling an activator group for the carbonyl group which is made to react with the product with the general formula IV, to be fixed is isobutyl chloroformate, and the operation is done in the presence of tri-n-butylamine, in anhydrous dioxane and in an inert atmosphere.

9) Process according to any one of the Claims 5 to 8, characterized in that the amino acid or its derivative which is made to react with the product with the general formula V is histamine marked with iodine $^{125}$ or $^{131}$, and that the operation is done in an inert atmosphere.

10) Use of the products as described in any one of the Claims 1 to 4 at the radio-immunological dosages of dienestrol, of hexestrol as well as of diethylstilbestrol, in the biological liquids and tissues of man or animals and in human and animal alimentation.

11) As new industrial products, the products with the general formula VI

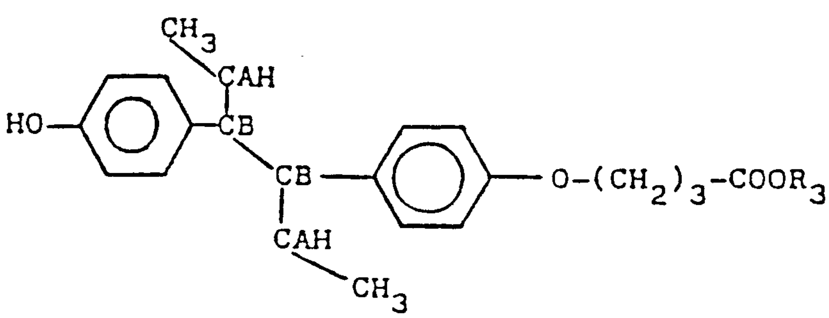

in which A and B each represent a hydrogen atom or together represent a double bond and $R_3$ represents a hydrogen atom, an alkyl radical having at the most 6 carbon atoms or an activator group of the carbonyl function, which is chosen from the group constituted by the group -CO-O-Alk, Alk being an alkyl radical having at the most 6 carbon atoms, or the groups resulting from the action of N-hydroxy succinimide or of dicyclohexylcarbodiimide on the carbonyl function.

12) Products according to Claim 11, namely,

- 4-0-monocarbethoxypropyl dienestrol with the formula:

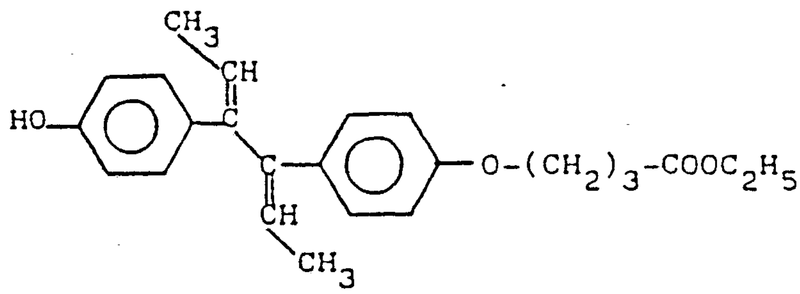

and 4-0-monocarbethoxypropyl hexestrol with the formula:

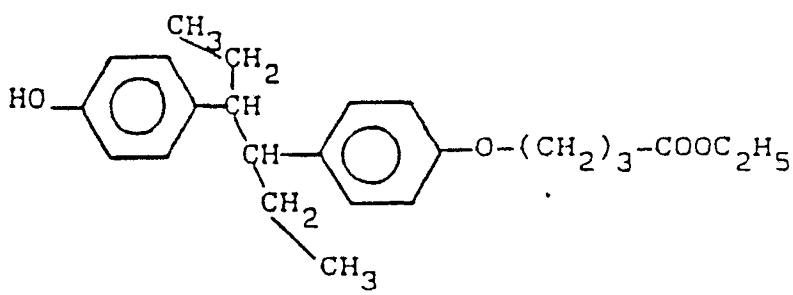

13) Products according to Claim 11, namely,
- 4-0-monocarboxypropyl dienestrol with the formula:

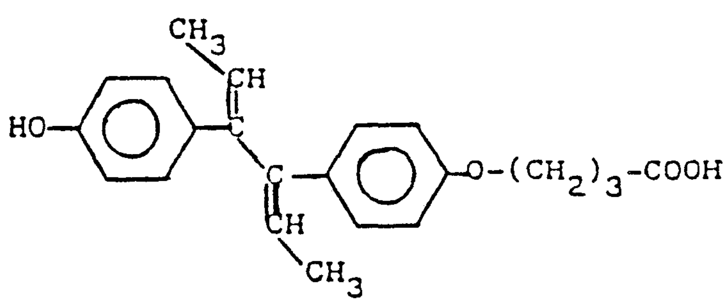

and 4-0-monocarboxypropyl hexestrol with the formula:

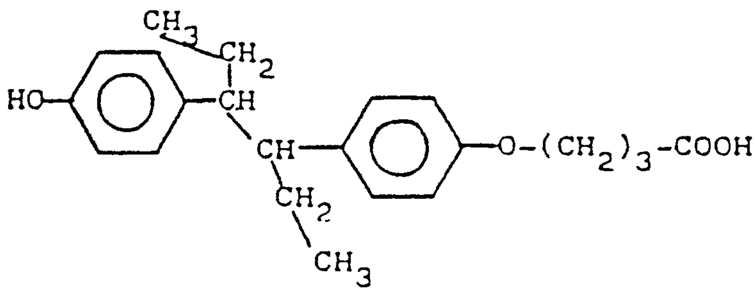

14) Products according to Claim 11, namely
- the mixed anhydride of 4-0-monocarboxypropyl dienstrol with isobutyl formate with the formula:

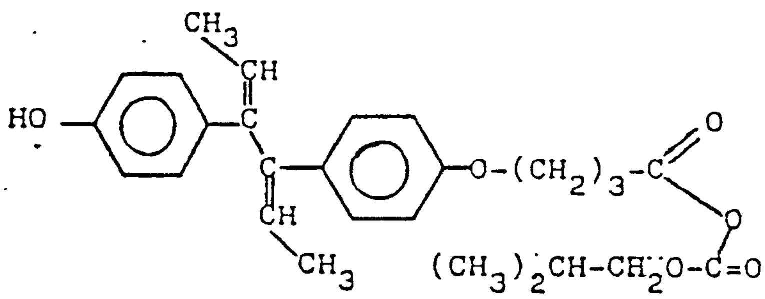

- the mixed anhydride of 4-0-monocarboxypropyl hexestrol with isobutyl formate with the formula:

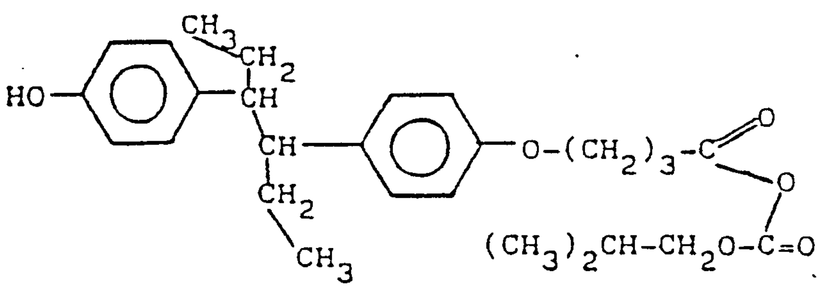

**Claims:** for the contracting stage AT

1) Process for the preparation of the derivatives of radioactive stilbene marked with iodine 125 or 131, with the general formula I:

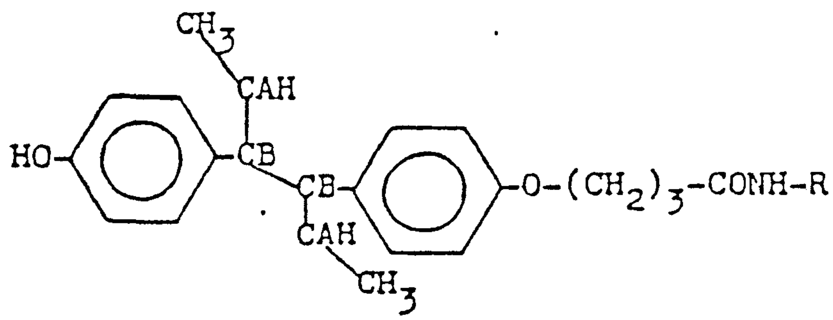

in which A and B each represent a hydrogen atom or together represent a double bond and R represents the residue of an amino acid $RNH_2$ or the residue of a derivative of this latter, this residue possessing an iodine acceptor group and being marked with iodine 125 or 131, characterized in that

- a product with the general formula II:

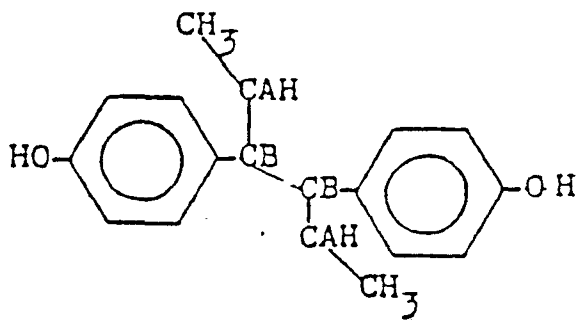

in which and in what follows, A and B have the previously stated significances, is made to react with an alkyl halogeno butyrate, in the presence of a base, so as to obtain the ether with the general formula III:

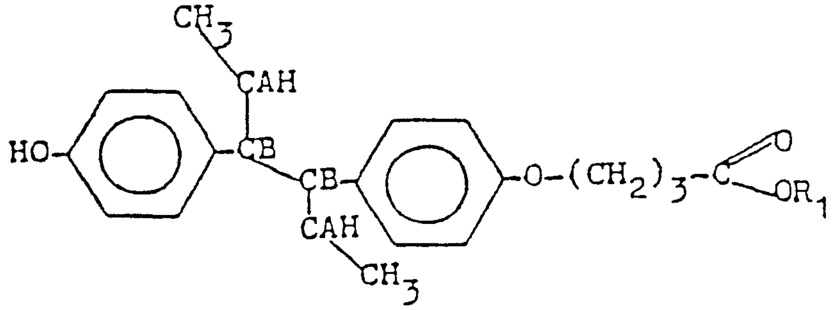

in which $R_1$ represents an alkyl radical having at the most 6 carbon atoms, of which the ester function is saponified,

so as to obtain a product with the general formula IV:

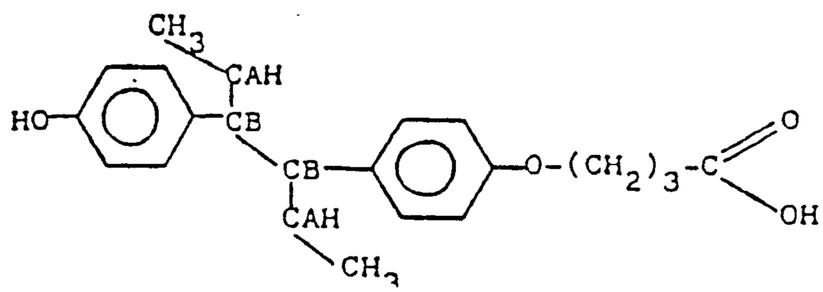

- then, on the acid function of this product with the formula IV an activator group for the carbonyl function is fixed and there is obtained the product with the general formula V:

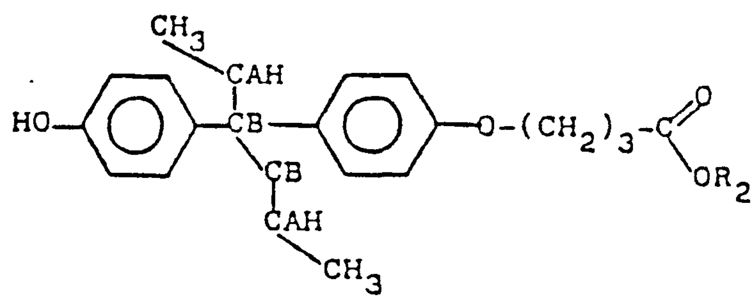

in which $R_2$ represents an activator group for the carbonyl function,

- which is made to react with an amino acid iodine acceptor or with a derivative of such an acid, and the product sought with the general formula I is obtained.

2) Process according to Claim 1, characterized in that the amino acid or the derivative of the amino acid iodine acceptor is chosen from the group constituted by histidine, tyrosine, histamine, tyramine and methyl tyrosinate.

3) Process according to Claim 1, characterized in that an activator group for the carbonyl function is fixed on the acid function of the product with the general formula IV by making the product with the formula IV react with an alkyl halogeno-formate, in the presence of a tertiary base, in an anhydrous medium and in an inert atmosphere, and so obtain the product with the general formula V

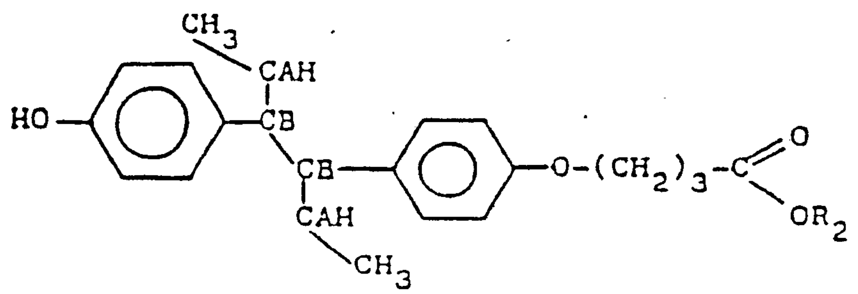

in which $R_2$ is an activator group of the carbonyl function, with the formula: -CO-O-Alk, Alk being an alkyl radical having at the most 6 carbon atoms.

4) Process according to any one of the Claims 1 to 3, characterized in that

- the alkyl halogenobutyrate which is made to react on the product with the general formula II is ethyl bromobutyrate and the operation is done in the presence of potassium carbonate
- the ester function of the compound with the general formula III is saponified by means of sodium hydroxide in methanol.

5) Process according to any one of the Claims 1 to 4, characterized in that the amino acid or its derivative which is made to react with the product with the general formula V is histamine, marked with iodine 125 or 131, and that the operation is done in an inert atmosphere.

6) Process according to any one of the Claims 1 to 5, characterized in that at the start dienestrol is used, and that 4-0-monocarboxypropyl dienestrol is obtained, coupled to ($^{125}$I)-histamine with the formula:

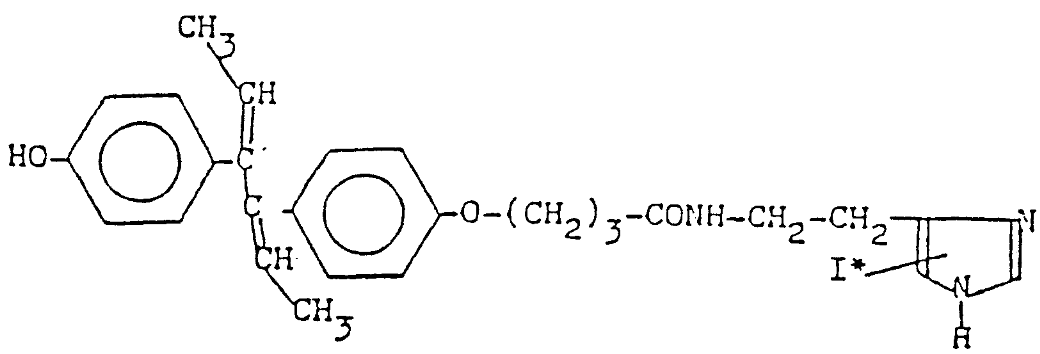

in which the sign * indicates that radioactive iodine$^{125}$ is concerned, which iodine$^{125}$ can be in position 2 or 5.

7) Process according to any one of the Claims 1 to 5, characterized in that at the start hexestrol is used and that 4-0-monocarboxypropyl hexestrol is obtained, coupled to ($^{125}$I)-histamine with the formula:

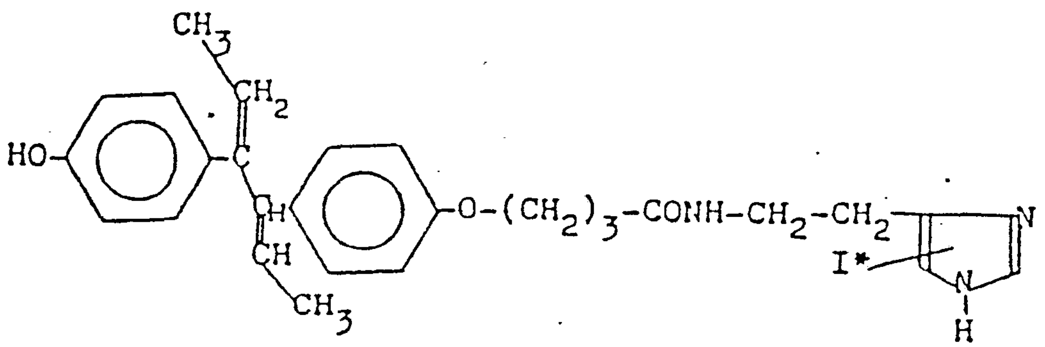

in which the sign * indicates that radioactive iodine[125] is concerned, whioh iodine [125] can be in position 2 or 5.

8) Use of the products as described in any one of the Claims 1 and 5 to 7, at the radio-immunological dosages of dienestrol, of hexestrol as well as of diethylstilbestrol, in the biological liquids and tissues of man or of animals and in human and animal alimentation.